# EUROPEAN PATENT SPECIFICATION

(11) **EP 4 281 433 B1**
(45) Date of publication and mention of the grant of the patent: **25.12.2024**
(21) Application number: 22718600.4
(22) Date of filing: 24.03.2022
(51) Int. Cl.: C07C 61/04, A61K 8/03

(54) **INCENSE ODORANT**
WEIHRAUCHDUFTSTOFF
ENCENS ODORANT

(30) Priority: 29.03.2021 EP 21165696
(43) Date of publication of application: 29.11.2023
(73) Proprietor: Firmenich SA, 1242 Satigny (CH)
(72) Inventor: CHAPUIS, Christian, 1242 Satigny (CH)
(74) Representative: Strych, Sebastian
(86) International application number: PCT/EP2022/057758
(87) International publication number: WO 2022/207448

(56) References cited:
- US-A1- 2018 163 157
- CERUTTI-DELASALLE CÉLINE ET AL: "The (+)- cis - and (+)- trans -Olibanic Acids: Key Odorants of Frankincense", vol. 55, no. 44, 4 October 2016 (2016-10-04), DE, pages 13719 - 13723, XP055837663, ISSN: 1433-7851, Retrieved from the Internet <URL:https://api.wiley.com/onlinelibrary/tdm/v1/articles/10.1002%2Fanie.201605242> DOI: 10.1002/anie.201605242
- BARAN MARZENA ET AL: "Development of small-molecule inhibitors of fatty acyl-AMP and fatty acyl-CoA ligases in Mycobacterium tuberculosis", EUROPEAN JOURNAL OF MEDICINAL CHEMISTRY, ELSEVIER, AMSTERDAM, NL, vol. 201, 13 June 2020 (2020-06-13), XP086233561, ISSN: 0223-5234, [retrieved on 20200613], DOI: 10.1016/J.EJMECH.2020.112408

## Description

### Technical field

The present invention relates to the field of perfumery. More particularly, it concerns a composition of matter comprising a compound of formula (I) as defined below wherein the weight ratio of the cis-diastereoisomers to the trans-diastereoisomers is comprised in the range between 90:10 and 99.5:0.5 and comprising at least 10% w/w of cis-(1S,2R) diastereoisomer. Said composition of matter is a useful perfumery ingredient, and therefore the present invention comprises the invention composition of matter as part of a perfuming composition or of a perfuming consumer product.

### Background of the invention

The perfumery industry is constantly on the lookout for new ingredients in order to support the creativity of perfumer by diversifying the notes present in their palette. Furthermore, perfumers seek powerful and tenacious perfuming ingredients allowing bringing to any compositions a typical character perceived during several hours. However, only a few ingredients imparting incense note are available. So there is a need to develop alternatives.

WO2016079431 and Angew. Chem. Int. Ed., 2016, 55, 13719-13723 reports, as a compound imparting said olfactive properties, the compound of formula (I), wherein n is 1. Most particularly the second prior art mentions that incense note is due to the two following diasteroiomers: cis-(1S,2R)-2-octylcyclopropanecarboxylic acid and trans-(1S,2S)-2-octylcyclopropanecarboxylic. However, it has been surprisingly now shown that the trans diastereoisomers have a negative impact on the organoleptic properties of compound of formula (I) and that cis-(1R,2S)-2-octylcyclopropanecarboxylic acid is also an important diastereoisomer.

US2018/0163157 A1 describes 2-octylcyclopropyl-1-carboxylic acid, in particular in the form of one of the isomers thereof in isolated form or in the form of a mixture of at least two of said isomers.

So, the present invention provides a novel composition of matter as defined above comprising mainly cis-diastereoisomers leading to a very powerful and substantive incense note combined with citrus aspect never reported in the prior art. The prior art does not anticipate that the present composition of matter provides such increase in performance while delivering a citrus note.

### Summary of the Invention

The invention relates to a composition of matter as defined above never disclosed imparting, in addition to incense odor note, a citrus odor note and its use as a perfuming ingredient.

So a first object of the present invention is a composition of matter comprising a compound of formula (I) 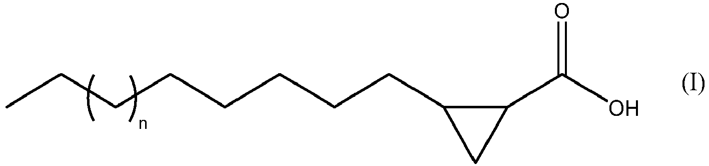 wherein n is 1 or 2; the weight ratio of the cis-diastereoisomers to the trans-diastereoisomers is comprised in the range between 90:10 and 99.5:0.5 and comprising at least 10% w/w of cis-(1S,2R) diastereoisomer, the percentage being relative to the total weight of the composition of matter.

A second object of the present invention is a method to confer, enhance, improve or modify the odor properties of a perfuming composition or of a perfumed article or of a surface, which method comprises adding to said composition or perfumed article an effective amount of a composition of matter as defined above.

Another object of the present invention is the use as perfuming ingredient of a composition of matter as defined above.

Another object of the present invention is a perfuming composition comprising
i) a composition of matter, as defined above;
ii) at least one ingredient selected from the group consisting of a perfumery carrier and a perfumery base; and
iii) optionally at least one perfumery adjuvant.

A further object of the present invention is a perfumed consumer product comprising a composition of matter as defined above or a perfuming composition as defined above.

### Description of the invention

A surprising synergic effect has been discovered between the 4 diastereoisomers of the compound of formula (I) leading to the invention composition of matter possessing a powerful and substantive incense note and surprisingly also imparting citrus aspect.

So, a first object of the present invention is a composition of matter comprising a compound of formula (I) 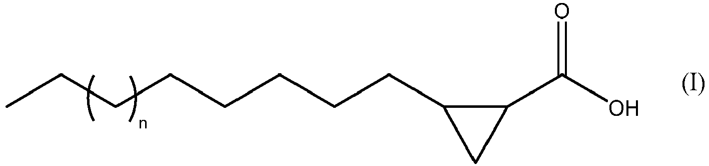 wherein n is 1 or 2; and the weight ratio of the cis-diastereoisomers to the trans-diastereoisomers is comprised in the range between 90:10 and 99.5:0.5 and comprising at least 10% w/w of cis-(1S,2R) diastereoisomer, the percentage being relative to the total weight of the composition of matter.

Said composition of matter can be used as perfuming ingredient, for instance to impart powerful and substantive odor notes of the incense type with citrus facet.

By the terms "cis-diastereoisomers" and "trans-diastereoisomers", it is meant the normal meaning understood by a person skilled in the art, i.e the cis-diastereoisomers correspond to cis-(1S,2R)-2-nonylcyclopropanecarboxylic acid and cis-(1R,2S)-2-nonylcyclopropanecarboxylic acid when n is 2 or cis-(1S,2R)-2-octylcyclopropanecarboxylic acid and cis-(1R,2S)-2-octylcyclopropanecarboxylic acid when n is 1 and trans-diastereoisomers correspond to trans-(1S,2S)-2-nonylcyclopropanecarboxylic acid and trans-(1R,2R)-2-nonylcyclopropanecarboxylic acid when n is 2 or trans-(1S,2S)-2-octylcyclopropanecarboxylic acid and trans-(1R,2R)-2-octylcyclopropanecarboxylic acid when n is 1.

By the term "cis-(1S,2R) diastereoisomer", it is meant the normal meaning understood by a person skilled in the art, i.e cis-(1S,2R)-2-nonylcyclopropanecarboxylic acid when n is 2 and cis-(1S,2R)-2-octylcyclopropanecarboxylic acid when n is 1.

According to any embodiments of the invention, the invention's composition of matter comprises at least 15% w/w, 20% w/w, 25% w/w, 30% w/w, 35% w/w, 40% w/w of cis-(1S,2R) diastereoisomer, the percentage being relative to the total weight of the composition of matter. In particular, the invention's composition of matter comprises at least 15% w/w, 20% w/w, 25% w/w, 30% w/w, 35% w/w, 40% w/w of cis-(1S,2R)-2-octylcyclopropanecarboxylic acid, the percentage being relative to the total weight of the composition of matter.

According to any embodiments of the invention, n is 1. In other words, the invention's composition of matter comprises:
a) from 90 to 99.5% w/w of cis-2-octylcyclopropanecarboxylic acid;
b) from 0.5% to 10% w/w of trans-2-octylcyclopropanecarboxylic acid;
the percentage being relative to the total weight of the composition of matter.

According to any embodiments of the invention, trans-2-octylcyclopropanecarboxylic acid may be in a form of a pure enantiomer or in the form of a mixture of two enantiomers. Trans-2-octylcyclopropanecarboxylic acid can be in a racemic form or scalemic form. Therefore, trans-2-octylcyclopropanecarboxylic acid may be trans-(1S,2S)-2-octylcyclopropanecarboxylic acid, trans-(1R,2R)-2-octylcyclopropanecarboxylic acid or a mixture thereof. Particularly, trans-2-octylcyclopropanecarboxylic acid may be in a racemic form; i.e. a mixture of trans-(1S,2S)-2-octylcyclopropanecarboxylic acid and trans-(1R,2R)-2-octylcyclopropanecarboxylic acid in a weight ratio of 50 : 50.

According to any embodiments of the invention, cis-2-octylcyclopropanecarboxylic acid may be in a form of a pure enantiomer or in the form of a mixture of two enantiomers. Cis-2-octylcyclopropanecarboxylic acid can be in a racemic form or scalemic form. Therefore, cis-2-octylcyclopropanecarboxylic acid may be cis-(1S,2R)-2-octylcyclopropanecarboxylic acid, cis-(1R,2S)-2-octylcyclopropanecarboxylic acid or a mixture thereof. Particularly, cis-2-octylcyclopropanecarboxylic acid may be in a racemic form; i.e. a mixture of cis-(1S,2R)-2-octylcyclopropanecarboxylic acid and cis-(1R,2S)-2-octylcyclopropanecarboxylic acid in a weight ratio of 50 : 50.

According to any embodiments of the invention, the invention composition of matter may comprise at least 45% w/w of cis-(1S,2R)-2-octylcyclopropanecarboxylic acid, the percentage being relative to the total weight of the composition of matter.

According to any embodiments of the invention, in the present composition of matter the various constituents mentioned above are present in the following amounts:
a) from 45 to 99.5% w/w of cis-(1S,2R)-2-octylcyclopropanecarboxylic acid;
b) at most 49,75% w/w of cis-(1R,2S)-2-octylcyclopropanecarboxylic acid;
c) at most 10% w/w of trans-(1S,2S)-2-octylcyclopropanecarboxylic acid;
d) at most 10% w/w of trans-(1R,2R)-2-octylcyclopropanecarboxylic acid.

the percentage being relative to the total weight of the composition of matter; and
wherein the weight ratio of the cis-diastereoisomers to the trans-diastereoisomers is comprised in the range between 90:10 and 99.5:0.5.

According to an embodiment of the invention, in the present composition of matter the various constituents mentioned above are present in the following amounts:
a) from 47.5 to 99% w/w of cis-(1S,2R)-2-octylcyclopropanecarboxylic acid;
b) at most 49.5% w/w of cis-(1R,2S)-2-octylcyclopropanecarboxylic acid;
c) at most 5% w/w of trans-(1S,2S)-2-octylcyclopropanecarboxylic acid;
d) at most 5% w/w of trans-(1R,2R)-2-octylcyclopropanecarboxylic acid.

According to any one of the above embodiments of the invention, the present composition of matter may comprise from about 45 to 95% w/w of (cis-(1S,2R)-2-octylcyclopropanecarboxylic acid, particularly from about 47.5 to 90% w/w of (cis-(1S,2R)-2-octylcyclopropanecarboxylic acid, particularly, from about 47.5 to 80% w/w of (cis-(1S,2R)-2-octylcyclopropanecarboxylic acid, particularly, from about 47.5 to 70% w/w of (cis-(1S,2R)-2-octylcyclopropanecarboxylic acid, particularly, from about 47.5 to 60% w/w of (cis-(1S,2R)-2-octylcyclopropanecarboxylic acid, and more particularly from about 47.5 to 50% w/w of (cis-(1S,2R)-2-octylcyclopropanecarboxylic acid.

According to a particular embodiment of the invention, the present composition of matter may comprise from about 1 to 49.75% w/w of cis-(1R,2S)-2-octylcyclopropanecarboxylic acid, particularly, from about 5 to 49.5% w/w of cis-(1R,2S)-2-octylcyclopropanecarboxylic acid, particularly, from about 10 to 49.5% w/w of cis-(1R,2S)-2-octylcyclopropanecarboxylic acid, particularly, from about 20 to 49.5% w/w of cis-(1R,2S)-2-octylcyclopropanecarboxylic acid, particularly, from about 30 to 49.5% w/w of cis-(1R,2S)-2-octylcyclopropanecarboxylic acid, particularly, from about 40 to 49.5% w/w of cis-(1R,2S)-2-octylcyclopropanecarboxylic acid, even more particularly, from about 45 to 49.5% w/w of cis-(1R,2S)-2-octylcyclopropanecarboxylic acid.

According to any one of the above embodiments of the invention, the present composition of matter may comprise from about 0.25 to 5% w/w of trans-(1S,2S)-2-octylcyclopropanecarboxylic acid, and more particularly from about 0.5 to 3% w/w of trans-(1S,2S)-2-octylcyclopropanecarboxylic acid.

According to any one of the above embodiments of the invention, the present composition of matter may comprise from about 0.25 to 5% w/w of trans-(1R,2R)-2-octylcyclopropanecarboxylic acid, and more particularly from about 0.5 to 3% w/w of trans-(1S,2S)-2-octylcyclopropanecarboxylic acid.

According to an embodiment of the invention, in the present composition of matter the various constituents mentioned above are present in the following amounts:
a) from 47.5 to 49.75% w/w of cis-(1S,2R)-2-octylcyclopropanecarboxylic acid;
b) from 47.5 to 49.75% w/w of cis-(1R,2S)-2-octylcyclopropanecarboxylic acid;
c) from 0.5% to 2.5% w/w of trans-(1S,2S)-2-octylcyclopropanecarboxylic acid;
d) from 0.5% to 2.5% w/w of trans-(1R,2R)-2-octylcyclopropanecarboxylic acid;

According to another particular embodiment of the invention, in the present composition of matter the various constituents mentioned above are present in the following amounts:
a) from 10 to 49,75% w/w of cis-(1S,2R)-2-octylcyclopropanecarboxylic acid;
b) from 45 to 99.5% w/w of cis-(1R,2S)-2-octylcyclopropanecarboxylic acid;
c) at most 10% w/w of trans-(1S,2S)-2-octylcyclopropanecarboxylic acid;
d) at most 10% w/w of trans-(1R,2R)-2-octylcyclopropanecarboxylic acid.

According to any one of the above embodiments of the invention, the weight ratio of the cis-diastereoisomers to the trans-diastereoisomers is comprised in the range between 92:8 and 99.5:0.5, more particularly in the range between 95:5 and 99:1; and more particularly in the range between 97:3 and 99: 1.

As mentioned above, the composition of matter of the invention possesses a very powerful natural incense organoleptic properties allied with a citrus character while possessing a long-lastingness. The overall odor profile is highly appreciated by perfumers since it opens up new directions in the perfumer's creativity when compared with the prior art ingredients.

Indeed, when the odor of the invention's composition of matter is compared with prior art composition of matter comprising more than 10% w/w of trans isomers, then the invention's compositions of matter distinguish themselves by a clearly different odor profile having citrus facet never reported while maintaining an intense and natural incense and lacking the fatty and dusty characteristic of the prior art compounds. Moreover, the invention's compositions of matter impart a clearly stronger and more substantive note while bringing more radiance to perfuming compositions. The invention's compositions of matter distinguish themselves also by showing a nicer olfactive profile.

Said differences lend the invention's compositions of matter and the prior art compounds to be each suitable for different uses, i.e. to impart different organoleptic impressions.

As mentioned above, the invention concerns the use of the invention's compositions of matter as a perfuming ingredient. In other words, it concerns a method or a process to confer, enhance, improve or modify the odor properties of a perfuming composition or of a perfumed article or of a surface, which method comprises adding to said composition or article an effective amount of the invention's composition of matter, e.g. to impart its typical note. Understood that the final hedonic effect may depend on the precise dosage and on the organoleptic properties of the invention's composition of matter, but anyway the addition of the invention's compositions of matter will impart to the final product its typical touch in the form of a note, touch or aspect depending on the dosage. In addition, the invention's composition of matter may also be used to enhance the organoleptic properties of perfuming ingredient or to decrease the unpleasant olfactory impression such as metallic, dusty or chemical facet of some perfuming ingredients.

By "use of the invention's compositions of matter it has to be understood here also the use of any composition containing the invention's compositions of matter and which can be advantageously employed in the perfumery industry.

Said compositions, which in fact can be advantageously employed as perfuming ingredients, are also an object of the present invention.

Therefore, another object of the present invention is a perfuming composition comprising:
i) as a perfuming ingredient, the invention's composition of matter as defined above;
ii) at least one ingredient selected from the group consisting of a perfumery carrier and a perfumery base; and
iii) optionally at least one perfumery adjuvant.

By "perfumery carrier" it is meant here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples, solvents such as butylene or propylene glycol, glycerol, dipropyleneglycol and its monoether, 1,2,3-propanetriyl triacetate, dimethyl glutarate, dimethyl adipate 1,3-diacetyloxypropan-2-yl acetate, diethyl phthalate, isopropyl myristate, benzyl benzoate, benzyl alcohol, 2-(2-ethoxyethoxy)-1-ethano, tri-ethyl citrate or mixtures thereof, which are the most commonly used. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company), or hydrogenated castors oils such as those known under the trademark Cremophor^{®} RH 40 (origin: BASF).

Solid carrier is meant to designate a material to which the perfuming composition or some element of the perfuming composition can be chemically or physically bound. In general such solid carriers are employed either to stabilize the composition, or to control the rate of evaporation of the compositions or of some ingredients. Solid carriers are of current use in the art and a person skilled in the art knows how to reach the desired effect. However by way of non-limiting examples of solid carriers, one may cite absorbing gums or polymers or inorganic materials, such as porous polymers, cyclodextrins, wood based materials, organic or inorganic gels, clays, gypsum talc or zeolites.

As other non-limiting examples of solid carriers, one may cite encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloide: Stabilisatoren, Dickungs- und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation techniques.

As non-limiting examples of solid carriers, one may cite in particular the core-shell capsules with resins of aminoplast, polyamide, polyester, polyurea or polyurethane type or a mixture threof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycoluryl, melamine, methylol melamine, methylated methylol melamine, guanazole and the like, as well as mixtures thereof. Alternatively one may use preformed resins alkylolated polyamines such as those commercially available under the trademark Urac^{®} (origin: Cytec Technology Corp.), Cymel^{®} (origin: Cytec Technology Corp.), Urecoll^{®} or Luracoll^{®} (origin: BASF).

Other resins are the ones produced by the polycondensation of a polyol, like glycerol, and a polyisocyanate, like a trimer of hexamethylene diisocyanate, a trimer of isophorone diisocyanate or xylylene diisocyanate or a Biuret of hexamethylene diisocyanate or a trimer of xylylene diisocyanate with trimethylolpropane (known with the tradename of Takenate^{®}, origin: Mitsui Chemicals), among which a trimer of xylylene diisocyanate with trimethylolpropane and a Biuret of hexamethylene diisocyanate are preferred.

Some of the seminal literature related to the encapsulation of perfumes by polycondensation of amino resins, namely melamine based resins with aldehydes includes articles such as those published by K. Dietrich et al. Acta Polymerica, 1989, vol. 40, pages 243, 325 and 683, as well as 1990, vol. 41, page 91. Such articles already describe the various parameters affecting the preparation of such core-shell microcapsules following prior art methods that are also further detailed and exemplified in the patent literature. US 4'396'670, to the Wiggins Teape Group Limited is a pertinent early example of the latter. Since then, many other authors have enriched the literature in this field and it would be impossible to cover all published developments here, but the general knowledge in encapsulation technology is very significant. More recent publications of pertinence, which disclose suitable uses of such microcapsules, are represented for example by the article of K. Bruyninckx and M. Dusselier, ACS Sustainable Chemistry & Engineering, 2019, vol. 7, pages 8041-8054.

By "perfumery base" what is meant here is a composition comprising at least one perfuming co-ingredient.

By "perfuming co-ingredient" it is meant here a compound, which is used in a perfuming preparation or a composition to impart a hedonic effect. In other words such a co-ingredient, to be considered as being a perfuming one, must be recognized by a person skilled in the art as being able to impart or modify in a positive or pleasant way the odor of a composition, and not just as having an odor. The perfuming co-ingredient may impart an additional benefit beyond that of modifying or imparting an odor, such as long-lasting, blooming, malodour counteraction, antimicrobial effect, antiviral effect, microbial stability, or pest control.

The nature and type of the perfuming co-ingredients present in the base do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin.

In particular, one may cite perfuming co-ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0^{2,7}]undecan-4-one, 1-methoxy-3-hexanethiol, 2-ethyl-4,4-dimethyl-1,3-oxathiane, 2,2,7/8,9/10-Tetramethylspiro[5.5]undec-8-en-1-one, menthol and/or alpha-pinene;
- Balsamic ingredients: coumarin, ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
- Floral ingredients:methyl dihydrojasmonate, linalool, citronellol, phenylethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, 1-(2,6,6-trimethyl-1,3-cyclohexadien-1-yl)-2-buten-1-one, (2E)-1-(2,6,6-trimethyl-2-cyclohexen-1-yl)-2-buten-1-one, (2E)-1-[2,6,6-trimethyl-3-cyclohexen-1-yl]-2-buten-1-one, (2E)-1-(2,6,6-trimethyl-1-cyclohexen-1-yl)-2-buten-1-one, 2,5-dimethyl-2-indanmethanol, 2,6,6-trimethyl-3-cyclohexene-I-carboxylate, 3-(4,4-dimethyl-1-cyclohexen-1-yl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 8-decen-5-olide, 4-phenyl-2-butanone, isononyle acetate, 4-(1,1-dimethylethyl)-1-cyclohexyl acetate, verdyl isobutyrate and/or mixture of methylionones isomers;
- Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, 3-(3,3/1,1-dimethyl-5-indanyl)propanal, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
- Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1 -(5,5 -dimethyl -1 -cyclohexen-1-yl)-4-penten-1-one;
- Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{1S)-1-[(1R)-3,3-dimethylcyclohexyl]ethoxy}-2-oxoethyl propionate 3-methyl-5-cyclopentadecen-1-one, 1,3,4,6,7,8-hexahydro-4,6,6,7,8,8-hexamethyl-cyclopenta-g-2-benzopyrane, (1S,1'R)-2-[1-(3',3'-dimethyl-1'-cyclohexyl)ethoxy]-2-methylpropyl propanoate, oxacyclohexadecan-2-oneand/or (1S,1'R)-[1-(3',3'-dimethyl-1'-cyclohexyl)ethoxycarbonyl]methyl propanoate, ;
- Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2.1.0^{2,7}]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, Clearwood^{®} (Origin: Firmenich SA,), (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3-methylpentan-2-ol, 1-(2,3,8,8-tetramethyl-1,2,3,4,6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonan and/or 3-(3-isopropyl-1-phenyl)butanal.

According to a particular embodiment, the invention's perfuming composition comprises, as a perfuming co-ingredient, at least one woody ingredient.

A perfumery base according to the invention may not be limited to the above mentioned perfuming co-ingredients, and many other of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds also known as properfume or profragrance. Non-limiting examples of suitable properfume may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, trans-3-(dodecylthio)-1-(2,6,6-trimethyl-3-cyclohexen-1-yl)-1-butanone, 3-(dodecyl sulfonyl)-1 -(2,6,6-trimethylcyclohex-3 -en-1 - yl)butan-1-one, a linear polysiloxane co-polymer of (3-mercaptopropyl)(methyl)dimethoxysilane, 2-(dodecylthio)octan-4-one, 2-(dodecylsulfonyl)octan-4-one, 4-oxooctan-2-yl dodecanoate, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2E,6Z)-2,6-nonadienyl hexadecanoate, (2E,6Z)-2,6-nonadien-1-yl tetradecanoate, (2E,6Z)-2,6-nonadien-1-yl dodecanoate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1 -(((Z)-hex-3 -en-1 -yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, 2-(1-phenethoxyprop-1-en-2-yl)naphthalene, (2-phenethoxyvinyl)benzene, 2-(1-((3,7-dimethyloct-6-en-1-yl)oxy)prop-1-en-2-yl)naphthalene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-methoxy-4-(1-phenethoxyprop-1-en-2-yl)benzene, (2-((2-methyl-4-(2,6,6-trimethylcyclohex-1-en-1-yl)but-1-en-1-yl)oxy)ethyl)benzene, 1-methoxy-4-(2-methyl-3-phenethoxyallyl)benzene, (2-((2-isopropyl-5-methylcyclohexylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 2-ethoxy-1-((2-methoxy-2-phenylvinyl)oxy)-4-methylbenzene, 3-methoxy-4-((2-methoxy-2-phenylvinyl)oxy)benzaldehyde, 1-isopropyl-2-((2-methoxy-2-phenylvinyl)oxy)-4-methylbenzene, 4-((2-(hexyloxy)-2-phenylvinyl)oxy)-3-methoxybenzaldehyde or a mixture thereof.

By "perfumery adjuvant", it is meant here an ingredient capable of imparting additional added benefit such as a color, a particular light resistance, chemical stability, etc. A detailed description of the nature and type of adjuvant commonly used in perfuming composition cannot be exhaustive, but it has to be mentioned that said ingredients are well known to a person skilled in the art. One may cite as specific non-limiting examples the following: viscosity agents (e.g. surfactants, thickeners, gelling and/or rheology modifiers), stabilizing agents (e.g. preservatives, antioxidant, heat/light and or buffers or chelating agents, such as BHT), coloring agents (e.g. dyes and/or pigments), preservatives (e.g. antibacterial or antimicrobial or antifungal or anti irritant agents), abrasives, skin cooling agents, fixatives, insect repellants, ointments, vitamins and mixtures thereof.

It is understood that a person skilled in the art is perfectly able to design optimal formulations for the desired effect by admixing the above mentioned components of a perfuming composition, simply by applying the standard knowledge of the art as well as by trial and error methodologies.

An invention's composition consisting of at least one composition of matter as defined above and at least one perfumery carrier consists of a particular embodiment of the invention as well as a perfuming composition comprising at least one composition of matter as defined above, at least one perfumery carrier, at least one perfumery base, and optionally at least one perfumery adjuvant.

For the sake of clarity, it is also understood that any mixture resulting directly from a chemical synthesis, e.g. a reaction medium without an adequate purification, in which the composition of matter of the invention would be involved as a starting, intermediate or end-product could not be considered as a perfuming composition according to the invention as far as said mixture does not provide the inventive composition of matter in a suitable form for perfumery. Thus, unpurified reaction mixtures are generally excluded from the present invention unless otherwise specified.

The invention's composition of matter can also be advantageously used in all the fields of modern perfumery, i.e. fine or functional perfumery, to positively impart or modify the odor of a consumer product into which said composition of matter is added. Consequently, another object of the present invention consists of a perfumed consumer product comprising, as a perfuming ingredient, at least one composition of matter, as defined above.

The invention's composition of matter can be added as such or as part of an invention's perfuming composition.

For the sake of clarity, "perfumed consumer product" is meant to designate a consumer product which delivers at least a pleasant perfuming effect to the surface or space to which it is applied (e.g. skin, hair, textile, or home surface). In other words, a perfumed consumer product according to the invention is a perfumed consumer product which comprises a functional formulation, as well as optionally additional benefit agents, corresponding to the desired consumer product, and an olfactive effective amount of at least one invention's composition of matter. For the sake of clarity, said perfumed consumer product is a non-edible product.

The nature and type of the constituents of the perfumed consumer product do not warrant a more detailed description here, which in any case would not be exhaustive, the skilled person being able to select them on the basis of his general knowledge and according to the nature and the desired effect of said product.

Non-limiting examples of suitable perfumed consumer products include a perfume, such as a fine perfume, a splash or eau de parfum, a cologne or a shave or after-shave lotion; a fabric care product, such as a liquid, a pod or solid detergent or tablet, a fabric softener, a liquid or solid scent booster, a dryer sheet, a fabric refresher, an ironing water, a paper, a bleach, a carpet cleaner, a curtain-care product; a body-care product, such as a hair care product (e.g. a shampoo, , a leave-on or rinse-off hair conditioner, a coloring preparation or a hair spray, a color-care product, a hair shaping product, a dental care product), a disinfectant, an intimate care product; a cosmetic preparation (e.g. a skin cream or lotion, a vanishing cream or a deodorant or antiperspirant (e.g. a spray or roll on), a hair remover, a tanning or sun or after sun product, a nail product, a skin cleansing, a makeup); or a skin-care product (e.g. a soap, a shower or bath mousse, oil or gel, or a hygiene product or a foot/hand care products); an air care product, such as an air freshener or a "ready to use" powdered air freshener which can be used in the home space (rooms, refrigerators, cupboards, shoes or car) and/or in a public space (halls, hotels, malls, etc..); or a home care product, such as a mold remover, a furnisher care product, a wipe, a dish detergent or a hard-surface (e.g. a floor, bath, sanitary or a window-cleaning) detergent; a leather care product; a car care product, such as a polish, a wax or a plastic cleaner.

Some of the above-mentioned perfumed consumer products may represent an aggressive medium for the invention's composition of matter, so that it may be necessary to protect the latter from premature decomposition, for example by encapsulation or by chemically binding it to another chemical which is suitable to release the invention's ingredient upon a suitable external stimulus, such as an enzyme, light, heat or a change of pH.

The proportions in which the composition of matter according to the invention can be incorporated into the various aforementioned products or compositions vary within a wide range of values. These values are dependent on the nature of the article to be perfumed and on the desired organoleptic effect as well as on the nature of the co-ingredients in a given base when the composition of matter according to the invention are mixed with perfuming co-ingredients, solvents or additives commonly used in the art.

For example, in the case of perfuming compositions, typical concentrations are in the order of 0.001 % to 30 % by weight, or even more, of the composition of matter of the invention based on the weight of the composition into which they are incorporated. In the case of perfumed consumer product, typical concentrations are in the order of 0.0001 % to 10 % by weight, or even more, of the composition of matter of the invention based on the weight of the consumer product into which they are incorporated.

### Examples

The invention will now be described in further detail by way of the following examples, wherein the abbreviations have the usual meaning in the art, the temperatures are indicated in degrees centigrade (°C). NMR spectra were acquired using either a Bruker Avance II Ultrashield 400 plus operating at 400 MHz, (¹H) and 100 MHz (¹³C) or a Bruker Avance III 500 operating at 500 MHz (¹H) and 125 MHz (¹³C) or a Bruker Avance III 600 cryoprobe operating at 600 MHz (¹H) and 150 MHz (¹³C). Spectra were internally referenced relative to tetramethyl silane 0.0 ppm. ¹H NMR signal shifts are expressed in δ ppm, coupling constants (*J*) are expressed in Hz with the following multiplicities: s, singlet; d, doublet; t, triplet; q, quartet; m, multiplet; b, broad (indicating unresolved couplings) and were interpreted using Bruker Topspin software. ¹³C NMR data are expressed in chemical shift δ ppm and hybridization from DEPT 90 and DEPT 135 experiments, C, quaternary (s); CH, methine (d); CH₂, methylene (t); CH₃, methyl (q). GC method GC-23 (20 M, 0.18 mm, 0.2um, H₂, 110°C at 3°C/min).

### Example 1

### Synthesis of the invention's composition of matter

### a) Step 1: preparation of ethyl 2-octylcycloprop-2-ene-1-carboxylate

A soln. of ethyl diazoacetate (85%/w in CH₂Cl₂, 3.24g, 24.11 mmol) in CH₂Cl₂ (35 ml) was added *via* a syringe pump in 12h to a mixture of 1-decyne (10.0 g, 72.3 mmol) and Rh₂(OAc)₄ (107 mg, 0.241 mmol). The solvent was removed in vacuo, and the residue was purified by CC/SiO₂ (cyclohexane/AcOEt 99:1 to 90:10) to afford ethyl 2-octylcycloprop-2-ene-1-carboxylate in 76% yield (based on the ethyl diazoacetate; 67% yield based on used/recuperated 1-decyne).

¹H-NMR: 6.32 (*s,* 1H); 4.18-4.10 (*m,* 2H); 2.49 (*dt*, *J* = 2, 7, 2H); 2.12 (*d*, *J* = 2, 1H); 1.60-1.54 (*m,* 2H); 1.38-1.33 (*m,* 2H); 1.37-1.23 (*m,* 8H); 1.26 (*t, J* = 7, 3H); 0.88 (*t, J* = 7, 3H). ¹³C-NMR: 176.7 (*s*); 115.7 (s); 93.9 (*d*); 60.1 (*t*); 31.8 (*t*); 29.3 (*t*); 29.2 (*t*); 29.1 (*t*); 26.7 (*t*); 25.0 (t); 22.7 (*t*); 19.7 (*d*); 14.4 (*q*); 14.1 (*q*).

### b) Step 2: preparation of 2-octylcycloprop-2-ene-1-carboxylic acid

LiOH (133 mg, 5.57 mmol) was added to a soln. of ethyl 2-octylcycloprop-2-ene-1-carboxylate (1000 mg, 4.46 mmol) in THF/H₂O 4:1 (15 ml), then the reaction mixture was heated to reflux for 56 h. The cold reaction mixture was concentrated in vacuo, diluted with H₂O (10 ml) and extracted with Et₂O (3 x 10 ml). The aq. phase was acidified to pH 1 with 35% aq. HCl, then re-extracted with Et₂O (3 x 15 ml). This org. phase was dried (Na₂SO₄), concentrated to afford quantitatively pure acid 2-octylcycloprop-2-ene-1-carboxylic acid. This latter was used crude, without distillation, for the next hydrogenation step.

¹H-NMR: 10.84 (brs, 1 OH); 6.32 (*s*, 1H); 2.51 (*tt*, *J* = 1, 7.8, 2H); 2.13 (*d*, *J* = 1, 1H); 1.60 (*dquint*, *J* = 2, 7.2, 2H); 1.39-1.33 (*m,* 2H); 1.32-1.22 (*m,* 8H); 0.88 (*t*, *J* = 7, 3H).

¹³C-NMR: 183.3 (*s*); 115.2 (*s*); 93.4 (*d*); 31.8 (*t*); 29.2 (2*t*); 29.1 (*t*); 26.6 (t); 24.9 (*t*) ; 22.6 (*t*); 19.5 (*d*); 14.1 (*q*).

### c) Step 3: preparation of the invention's composition of

A soln. of 2-octylcycloprop-2-ene-1-carboxylic acid (210 mg, 1.07 mmol) in AcOEt (10 ml) was hydrogenated over *Lindlar* cat (2.2 mg, 5% Pd/CaCO₃) for 3h (theor. 24 ml H₂). After full conversion, the reaction mixture was filtered through a short path of Celite^{®}, then concentrated in vacuo and bulb-to-bulb distilled to afford quantitatively a mixture comprising 98% of cis-(1SR,2RS)-2-octylcyclopropanecarboxylic acid and 2% of trans-(1SR,2SR)-2-octylcyclopropanecarboxylic acid.

Bp 150°C/0.40 mbar.

Analyses in accord with those obtained for the optically active version, as described in Example 3.

### Example 2

### Synthesis of the invention's composition of matter

### a) Step 1: preparation of ethyl 2-octylcyclopropane-1-carboxylate

A soln. of ethyl 2-octylcycloprop-2-ene-1-carboxylate ester (1.8g, 8.02 mmol) in AcOEt (32 ml) was hydrogenated over *Lindlar* cat (85 mg, 5% Pd/CaCO₃) for 3h (theor. 176 ml H₂). After full conversion, the reaction mixture was filtered through a short path of Celite^{®}, then concentrated in vacuo and bulb-to-bulb distilled to afford quantitatively ethyl 2-octylcyclopropane-1-carboxylate with a cis : trans ratio of 98 : 2.

Bp: 150°C/0.35 mbar.

¹H-NMR: 4.13 (*q, J* = 7, 2H); 1.66 (*dt*, *J* = 4.7, 8.7, 1H); 1.57-1.52 (*m,* 1H); 1.49-1.42 (*m,* 1H); 1.38-1.33 (*m,* 1H); 1.31-1.24 (*m,* 14H); 1.24-1,19 (*m,* 1H); 0.99 (*dt*, *J* = 4.7, 8, 1H); 0.92 (*dt, J* = 4.7, 6.7, 1H); 0.88 (*t*, *J* = 7, 3H).

¹³C-NMR: 173.1 (*s*); 60.2 (*t*); 31.9 (*t*); 29.7 (*t*); 29.6 (*t*); 29.4 (*t*) ; 29.3 (*t*) ; 27.0 (*t*) ; 22.7 (*t*) ; 22.0 (*d*) ; 18.2 (*d*); 14.4 (*q*); 14.1 (*q*); 13.3 (*t*).

### b) Step 2: preparation of the invention's composition of matter

A 10% aq. LiOH (5 ml) was added to a soln. of ethyl cis-2-octylcyclopropane-1-carboxylate (600 mg, 2.86 mmol) in MeOH (5 ml) at 20°C. The reaction mixture was stirred at this temperature for 12h, then acidified with 2N aq. HCl, and extracted with Et₂O. The organic layer was dried (Na₂SO₄), concentrated to afford a mixture comprising 98% of cis-(1SR,2RS)-2-octylcyclopropanecarboxylic acid and 2% of trans-(1SR,2SR)-2-octylcyclopropanecarboxylic acid in 87% yield. Analyses in accord with those obtained for the optically active version, as described in Example 3.

### Example 3

### Synthesis of the invention's composition of matter

Obtained from the known undec-2-yn-1-ol [Synthesis 1999, 107] according to the procedure published *in* Angew. Chem. Int. Ed. 2016, 55, 13719. Then purification by CC/SiO₂ (cyclohexane/AcOEt 99/1 to 9/1) to reach a stereochemical purity of 92% (+)-cis-(1S,2R)-2-octylcyclopropanecarboxylic acid and 8% (-)-trans-(1R,2R)-2-octylcyclopropanecarboxylic acid. R_{f} = 0.42 (c-hexane/AcOEt 7:3). [α]_{D}²⁰ = +29.1 (c = 0.036, CHCl₃). ¹H-NMR: 11.80 (brs, 1 OH); 1.68 (*dt*, *J* = 5.4, 8.2, 1H); 1.55 (*hept*, *J* = 7.3, 2H); 1.41-1.23 (*m,* 13H); 1.08 (*dt*, *J* = 4.1, 7.9, 1H); 0.965 (*dt*, *J* = 5.1, 7.6, 1H); 0.88 (*t*, *J* = 7, 3H). ¹³C-NMR: 180.0 (*s*); 31.9 (*t*); 29.6 (*t*); 29.5 (*t*); 29.3 (2*t*); 26.9 (*t*); 23.2 (*d*); 22.7 (*t*); 18.1 (*d*); 14.5 (*t*); 14.1 (*q*).

### Example 4

### Synthesis of the invention's composition of matter

### a) Step 1: preparation of (+-)-ethyl 2-nonyl-2-cyclopropene-l-carboxylate

A soln. of ethyl diazoacetate (85%/w in CH₂Cl₂, 2.97 g, 21.9 mmol) in CH₂Cl₂ (27 ml) was added via a syringe pump in 12h to a mixture of 1-undecyne (10.0 g, 65.67 mmol) and Rh₂(OAc)₄ (96 mg, 0.44 mmol). After 3 h at 20°C, the solvent was removed in vacuo, and the residue was purified by CC/SiO₂ (cyclohexane/AcOEt 99:1 to 90:10) to afford the desired (+-)-ethyl 2-nonyl-2-cyclopropene-1-carboxylate in 83% yield (based on the ethyl diazoacetate; 87% yield based on used/recuperated 1-undecyne).

¹H-NMR: 6.32 (br*q*, J = 1.5, 1H); 4.13 (*dq*, *J* = 4.0, 7.0, 2H); 2.49 (*t, J* = 7.1, 2H); 2.12 (*d*, *J* = 1.5, 1H); 1.58 (*quint*, *J* = 7.1, 2H); 1.37-1.31 (*m*, 2H); 1.30-1.26 (*m,* 10H); 1.25 (*t, J* = 7.1, 3H); 0.88 (*t*, *J* = 7.0, 3H).

¹³C-NMR: 176.7 (*s*); 115.7 (*s*); 93.9 (*d*); 60.1 (*t*); 31.9 (*t*); 29.5 (*t*); 29.3 (2*t*); 29.1 (*t*); 26.7 (*t*); 25.0 (*t*); 22.7 (*t*); 19.7 (*d*); 14.4 (*q*); 14.1 (*q*).

### b) Step 2: preparation of ethyl 2-nonylcyclopropanecarboxylate

A soln. of (+-)-ethyl 2-nonyl-2-cyclopropene-1-carboxylate (4.0 g, 16.78 mmol) in AcOEt (65 ml) was hydrogenated over *Lindlar* cat (170 mg, 5% Pd/CaCO₃) for 3h (theor. 368 ml H₂). After full conversion, the reaction mixture was filtered through a short path of *Celite*^{®}, then concentrated in *vacuo* and bulb-to-bulb distilled to afford quantitatively the saturated ethyl 2-nonylcyclopropanecarboxylate with a cis : trans ratio of 98 : 2. Bp: 150°C/0.25 mbar.

¹H-NMR: 4.13 (*q*, *J* = 7.0, 2H); 1,66 (*dt*, *J* = 5.4, 7.5, 1H); 1.58-1.52 (*m,* 1H); 1.49-1.43 (*m,* 1H); 1.37-1.32 (*m,* 1H); 1.31-1.23 (*m,* 14H); 1.26 (*t, J* = 7.0, 3H); 0.99 (*dt*, *J* = 5.0, 8.2, 1H); 0.93-0.90 (*m,* 1H); 0.88 (*t, J* = 7.0, 3H).

¹³C-NMR: 173.1 (*s*); 60.2 (*t*); 31.9 (*t*); 29.7 (2*t*); 29.6 (*t*); 29.4 (2*t*); 27.1 (*t*); 22.7 (*t*); 22.0 (*d*); 18.2 (*d*); 14.4 (*q*); 14.1 (*q*) ; 13.3 (*t*).

### c) Step 3: preparation of (+-)-2-nonyl-2-cyclopropene-1-carboxylic acid

LiOH (78 mg, 3.25 mmol) was added to a soln. of (+-)-ethyl 2-nonyl-2-cyclopropene-1-carboxylate (310 mg, 1.3 mmol) in THF/H₂O 4:1 (5 ml), then the reaction mixture was heated to reflux for 56 h. The cold reaction mixture was concentrated in *vacuo,* diluted with H₂O (5 ml) and extracted with Et₂O (3 x 10 ml). The aq. Phase was acidified to pH 1 with 35% aq. HCl, then re-extracted with Et₂O (3 x 15 ml). This org. phase was dried (Na₂SO₄), concentrated to afford quantitatively pure (+-)-2-nonyl-2-cyclopropene-1-carboxylic acid. This latter was used crude, without distillation, for the next hydrogenation step.

¹H-NMR: 10.90 (brs, 1H) ; 6.32 (br*q*, *J* = 1.5, 1H); 2.50 (br*t*, *J* = 7.0, 2H); 2.12 (*d*, *J* = 1.5, 1H); 1.59 (br*quint*, *J* = 7, 2H); 1.38-1.32 (*m,* 2H); 1.31-1.28 (*m,* 2H); 1.28-1.23 (*m,* 8H); 0.88 (*t*, *J* = 7.0, 3H).

¹³C-NMR: 183.4 (*s*); 115.2 (*s*); 93.4 (*d*); 31.9 (*t*); 29.5 (*t*); 29.3 (2*t*); 29.1 (*t*); 26.6 (*t*); 24.9 (*t*); 22.7 (*t*); 19.5 (*d*); 14.1 (*q*).

### d) Step 4 : preparation of (1RS,2SR)-2-nonylcyclopropane-1-carboxylic acid

A 10% aq. LiOH (5 ml) was added to a soln. of (1RS,2SR)-2-nonylcyclopropanecarboxylate (684 mg, 2.85 mmol) in MeOH (5 ml) at 20°C. The reaction mixture was stirred at this temperature for 18h, then acidified with 2N aq. HCl, and extracted with Et₂O. The org layer was dried (Na₂SO₄), concentrated to afford a mixture comprising 98% of cis-(1RS,2SR)-2-nonylcyclopropane-1-carboxylic acid and 2% of trans-(1RS,2RS)-2-nonylcyclopropanecarboxylic acid in 88% yield.

Also obtained quantitatively by *Lindlar* hydrogenation of (+-)-2-nonyl-2-cyclopropene-1-carboxylic acid, according to the conditions described above. Bp 165°C/0.05 mbar.

¹H-NMR: 11.74 (brs, 1H); 1.675 (*dt*, *J* = 5.4, 7.5, 1H); 1.55 (br*quint*, *J* = 7.0, 2H); 1.41-1.35 (*m,* 2H); 1.33-1.28 (*m,* 4H); 1.28-1.23 *(m,* 9H); 1.07 (*dt*, *J* = 4.1, 1H); 0.965 (*dt*, *J* = 5.2, 7.4, 1H); 0.88 (*t*, *J* = 7.0, 3H).

¹³C-NMR: 180.0 (*s*), 31.9 (*t*); 29.6 (*t*); 29.5 (2*t*); 29.3 (2*t*); 26.9 (*t*); 23.2 (*d*); 22.7 (*t*); 18.1 (*d*); 14.5 (*t*); 14.1 (*q*).

### Example 5

### Synthesis of a comparative composition of matter

### a) Step 1: preparation of undeca-1,3-dien-1-yl acetate

A E/Z mixture of commercially available 1-undec-2-enal (10.0 g, 59.4 mmol), AcOK (4.96 g, 50.5 mmol) in Ac₂O (15.14 ml, 16.38 g, 160 mmol) was refluxed for 4 h. The cold reaction mixture was poured onto ice/H₂O ( (100 ml). Et₂O (150 ml) was added and the partitioned org. phase was washed with satd. aq. NaHCO₃ until neutrality (solid NaHCO₃ was also added), then brine. The org. phase was dried (Na₂SO₄), concentrated, and the resulting oil was purified by bulb-to-bulb distillation to afford undeca-1,3-dien-1-yl acetate in 78% yield as a *ca.* 15:35:20:30 mixture of stereoisomers.

Bp: 130°C/1.3 mbar.

¹H-NMR of the mixture: 7.37-6.94 (*m,* 1H); 6.39-6.28 (*m,* 1H); 6.02-5.89 (*m,* 1H); 5.76-5.44 (*m,* 1H); 2.18 (*s*, 0.45H); 2.17 (*s*, 0.6H); 2.14 (*s*, 1.05H); 2.12 (*s*, 0.9H); 2.16-2.06 (*m,* 2H); 1.41-1.35 (*m,* 2H); 1.32-1.23 (*m,* 8H); 0.88 (*t*, *J* = 7, 3H).

¹³C-NMR (tentatively deduced from the mixture): major 167.9 (*s*); 138.2 (*d*); 132.9 (*d*); 122.7 (*d*); 111.3 (*d*); 31.9 (*t*); 29.3 (2*t*); 29.2 (2*t*); 22.7 (*t*); 20.7 (*q*); 14.1 (*q*); main medium 168.0 (*s*); 136.8 (*d*); 135.3 (*d*); 124.3 (*d*); 115.9 (*d*); 31.9 (*t*); 29.3 (2*t*); 27.8 (2*t*); 22.7 (*t*); 20.7 (*q*); 14.1 (*q*); minor medium 167.6 (*s*); 133.9 (*d*); 133.5 (*d*); 120.1 (*d*); 108.4 (*d*); 32.9 (*t*); 29.6 (2*t*); 29.2 (2*t*); 22.7 (*t*); 20.7 (*q*); 14.1 (*q*); minor 167.6 (*s*); 135.9 (*d*); 132.2 (*d*); 122.0 (*d*); 113.3 (*d*); 33.0 (*t*); 29.5 (2*t*); 27.7 (2*t*); 22.7 (*t*); 20.7 (*q*); 14.1 (*q*).

### b) Step 2: preparation of (Z)-undec-2-en-1-yl acetate

Undeca-1,3-dien-1-yl acetate (1.0 g, 4.75 mmol), maleic acid (34 mg, 0.295 mmol), [(Cp*)Ru(1,3-COD)][BF₄] (72 mg, 0.166 mmol), and acetone (6 ml) were charged in an autoclave under N₂. The autoclave was purged and then pressurized with H₂. After 18h at 60°C/5 bar, the autoclave was cooled down, depressurized and the reaction mixture was diluted with AcOEt (15 ml). Filtration over SiO₂ (10 g) and concentration afforded an oil which was purified by CC/SiO₂ (25 g, cyclohexane/AcOEt 99:1) to afford (Z)-undec-2-en-1-yl acetate in 46% yield as a 87:13 mixture of Z/E stereoisomer.

Rf = 0.22 (99:1).

¹H-NMR: (Z)-isomer 5.69-5.61 (*m,* 1H); 5.56-5.50 (*m,* 1H); 4.62 (*d*, *J* = 7.5, 2H); 2.10 (*q*, *J* = 7, 2H); 2.06 (*s*, 3H); 1.40-1.32 (*m,* 2H); 1.31-1.25 (*m,* 10H); 0.88 (*t, J* = 7, 3H). (*E*)-isomer tentatively deduced from the mixture 5.80-5.34 (*m,* 2H); 4.51 (*d*, *J* = 6.8, 2H); 2.11-2.03 (*m,* 2H); 2.04 (*s*, 3H); 1.41-1.32 (*m,* 2H); 1.31-1.25 (*m,* 10H); 0.88 (*t*, *J* = 7, 3H).

¹³C-NMR: (*Z*)-isomer 171.0 (*s*); 135.5 (*d*); 123.2 (*d*); 60.4 (*t*); 31.9 (*t*) ; 29.4 (*t*) ; 29.3 (2*t*) ; 29.2 (*t*) ; 27.6 (*t*); 22.7 (*t*); 21.0 (*q*); 14.1 (*q*). (*E*)-isomer tentatively deduced from the mixture 170.3 (*s*); 136.7 (*d*); 123.7 (*d*); 65.4 (*t*); 32.3 (*t*); 31.8 (*t*); 29.4 (*t*); 29.2 (*t*); 29.1 (*t*); 28.6 (*t*); 22.6 (*t*); 20.7 (*q*); 14.0 (*q*).

### c) Step 3: preparation (Z)-undec-2-en-1-ol

(Z)-undec-2-en-1-yl acetate (30 mg, 0.141 mmol) in MeOH (15 ml) was saponified with KOH (9.51 mg, 0.17 mmol) at 70°C for 3 h. The cold reaction mixture was concentrated under vacuo, and the residue was partitioned between Et₂O (10 ml) and H₂O (10 ml). The org. phase was washed with brine, dried (Na₂SO₄), then concentrated. Purification by CC/SiO₂ (cyclohexane/AcOEt 99:1 to 9:1) afforded quantitatively (Z)-undec-2-en-1-ol as a 87:13 mixture of Z/E stereoisomer.

¹H-NMR: (*Z*)-isomer 5.63-5.50 (*m,* 2H); 4.20 (br*t*, *J* = 4, 2H); 2.07 (*q, J* = 6.4, 2H); 1.66 (br*s*, 1 OH); 1.39-0.86 (*m,* 2H); 1.31-1.24 (*m,* 10H); 0.88 (*t, J* = 6.7, 3H). (*E*)-isomer 5.74-5.60 (*m,* 2H); 4.09 (*d*, *J* = 5.2, 2H); 2.04 (*q*, *J* = 7.3, 2H); 1.47 (br*s*, 1 OH); 1.41-1.33 (*m,* 2H); 1.32-1.24 (*m,* 10H); 0.88 (*t*, *J* = 6.7, 3H).

¹³C-NMR: (*Z*)-isomer 133.3 (*d*); 128.3 (*d*); 58.5 (*t*); 31.9 (*t*); 29.6 (*t*); 29.5 (*t*); 29.3 (2*t*); 27.5 (*t*); 22.7 (*t*); 14.1 (*q*). (*E*)-isomer 133.6 (*d*); 128.8 (*d*); 63.7 (*t*); 32.2 (*t*); 31.9 (*t*); 29.5 (*t*); 29.3 (*t*); 29.2 (2*t*); 22.7 (*t*); 14.1 (*q*).

### d) Step 4: preparation of cis-2-octylcyclopropyl-1-methanol

Et₂Zn (1M/hexane, 10 ml, 10 mmol) was added to a soln. of 87:13 (*Z*/*E*)-undec-2-en-1-ol (332 mg, 1.95 mmol) in hexane (10 ml) at -50°C, followed by CH₂I₂ (5.28g, 19.7 mmol). The reaction mixture was slowly equilibrated at 20°C and after 18h is cooled down again at -40°C. Sat. aq. NH₄Cl (20 ml) is added dropwise and the reaction mixture is equilibrated to 20°C. After partition, the aq. phase is washed with Et₂O (2 x 25 ml). The combined org. phases are washed with brine, dried (Na₂SO₄), concentrated, and the resulting orange oil is purified by CC/SiO₂ (cyclohexane/Et₂O 98:2 to 8:2) to afford cis-2-octylcyclopropyl-1-methanol (83% yield) as a colorless 84:16 cis/trans mixture.

Eventually, further purification by CC/SiO₂ (cyclohexane/AcOEt 99/1 to 9/1) then bulb-to-bulb distillation afforded pure 2-octylcyclopropyl-1-methanol with a cis : trans ratio of 98 : 2 in 64% yield.

Rf = 0.18 (9:1). Bp: 125°C/0.66 mbar.

¹H-NMR: *cis*-isomer3.64 (*dd, J* = 7, 11.3, 1H); 3.58 (*dd*, *J* = 8, 11.3, 1H); 1.58 (brs, 1 OH); 1.48-1.36 (*m,* 4H); 1.33-1.20 (*m,* 10H); 1.13-1.06 (*m,* 1H); 0.88 (*t, J* = 6.7, 3H); 0.87-0.83 (*m,* 1H); 0.70 (*dt*, *J* = 4.9, 8, 1H); -0.40 (*q*, *J* = 4.9, 1H). *trans*-isomer 3.44 (*dq*, *J* = 5.9, 10.6, 2H); 1.47 (br*s*, 1 OH); 1.36 (*q*, *J* = 7, 2H); 1.32-1.20 (*m,* 12H); 0.88 (*t, J* = 6.7, 3H); 0.86-0.80 (*m,* 1H); 0.62-0.56 (*m,* 1H); 0.36 (*dt*, *J* = 4.8, 9.2, 1H); 0.30 (*dt*, *J* = 4.8, 8.2, 1H). ¹³C-NMR: *cis*-isomer 63.3 (*t*); 31.9 (*t*); 30.2 (*t*); 29.6 (2*t*); 29.4 (*t*); 28.6 (*t*); 22.7 (*t*); 18.2 (*d*); 16.2 (*d*); 14.1 (*q*); 9.5 (*t*). *trans*-isomer 67.2 (*t*); 33.6 (*t*); 31.9 (*t*); 29.6 (2*t*); 29.5 (*t*); 29.4 (*t*); 22.7 (*t*); 21.2 (*d*); 17.2 (*d*); 14.1 (*q*); 9.9 (*t*).

### e) Step 5: preparation of the comparative composition of matter

*Jones* reagent (CrO₃, 2.7M in 36% H₂SO₄, 43.3 ml, 117 mmol) was added dropwise to a soln. of 2-octylcyclopropyl-1-methanol alcohol (8.7 g, 46.7 mmol 84:16 *cis*/*trans*) in acetone (100 ml) at 0°C. After 18h at 20°C, cold H₂O (100 ml) was added and the mixture was extracted with pentane (3 x 50 ml). The org. phase was extracted with 15% NaOH (100 ml). This basic aq. phase was acidified with 10% aq. HCl, and partitioned with pentane (3 x 100 ml). This org. phase was washed with brine (2 x 50 ml), dried (Na₂SO₄), filtered, concentrated to afford the acid mixture in 70% yield (84:16 cis-(1SR,2RS)-2-octylcyclopropanecarboxylic acid / trans-(1RS,2RS)-2-octylcyclopropanecarboxylic acid). Analyses in accord with those obtained for the optically active version, as described in Example 3.

### Example 6

### Synthesis of a comparative composition of matter

### a) Step 1: preparation of ethyl undeca-2,3-dienoate

Et₃N (4.4 ml, 31.6 mmol) was added at 0°C to a soln. of ethyl 2-(triphenylphosphoranylidene)acetate (10g, 28.7 mmol) in CH₂Cl₂ (80 ml). After 5 min. at 0°C, a soln. of nonanoyl chloride (6.59 g, 37.3 mmol) in CH₂Cl₂ (20 ml) was added dropwise at 0°C. After 18h at 20°C, the solvent was concentrated under vacuum. The residue was diluted with Et₂O (80 ml), then filtered. This process was repeated four times to precipitate Ph₃PO. A further purification over SiO₂ (80g, cyclohexane/AcOEt 99:1 to 8:2) afforded pure ethyl undeca-2,3-dienoate in 72% yield.

¹H-NMR: 5.605 (*q, J* = 6.6, 1H); 5.57 (*hex*, *J* = 3, 1H); 4.19 (*dq, J* = 2, 7, 2H); 2.13 (*dq*, *J* = 2, 7, 2H); 1.46 (*quint*, *J* = 7.2, 2H); 1.38-1.29 (*m,* 8H); 1.28 (*t, J* = 7, 3H); 0.88 (*t, J* = 7, 3H).

¹³C-NMR: 212.3 (*s*); 166.3 (*s*); 95.4 (*d*); 88.2 (*d*); 60.7 (*t*); 31.8 (*t*); 29.0 (*t*); 28.9 (*t*); 28.7 (*t*); 27.5 (*t*); 22.6 (*t*); 14.3 (*q*); 14.1 (*q*).

### b) Step 2: preparation of undeca-2,3-dien-1-ol

A soln. of ethyl undeca-2,3-dienoate ester (1.3g, 5.19 mmol) in CH₂Cl₂ (10 ml) was added dropwise in 30 min. to a soln. of DIBAH (1.2 M in toluene, 9.09 ml, 10.9 mmol) in CH₂Cl₂ (15 ml) at 0°C. After 2h, MeOH (10 ml) was added carefully, followed by 3N HCl. The aq. phase was extracted with Et₂O. The org. phase was washed with 10% NaOH, dried (Na₂SO₄), filtered, and concentrated under vacuum to afford in 45% yield pure undeca-2,3-dien-1-ol (72% yield when used without further purification in the next step).

¹H-NMR: 5.34-5.27 (*m,* 2H); 4.11 (br*s*, 2H); 2.02 (*dq*, *J* = 2.6, 7.4, 2H); 1.59 (brs, 1 OH); 1.41 (*quint*, *J* = 7.4, 2H); 1.34-1.23 (*m,* 8H); 0.88 (*t, J* = 7, 3H).

¹³C-NMR: 203.0 (s); 94.1 (*d*); 91.8 (*d*); 60.8 (*t*); 31.9 (*t*); 29.1 (3*t*); 28.7 (*t*); 22.7 (*t*); 14.1 (*q*).

### c) Step 3: preparation of (2-octylidenecyclopropyl)methanol

Et₂Zn (1M in hexane, 1.41 ml, 1.41 mmol) was added to ClCH₂CH₂Cl (5 ml) and this soln. was cooled down to -15°C. CH₂I₂ (473 mg, 1.76 mmol) in ClCH₂CH₂Cl (5 ml) was added dropwise in 1h *via* a syringe pump, while a white precipitate was formed. After an additional 30 min at - 15°C, a soln. of undeca-2,3-dien-1-ol (250 mg, 1.41 mmol) in ClCH₂CH₂Cl (4 ml) was added dropwise in 30 min. After an additional period of 3h at - 15°C, the temp was equilibrated to -10°C, then slowly to 20°C. The white soln. was poured carefully into sat. aq. NH₄Cl, then partitioned with Et₂O. The org. phase was washed with 5% HCl, then aq. sat. NaHCO₃, then H₂O, and dried (Na₂SO₄). Concentration afforded a 66:33 mixture of stereoisomers. Further purification over SiO₂/AgNO₃ with cyclohexane/AcOEt 99:1 to 9:1) afforded each of the diastereoisomers in 26% yield, respectively.

¹H-NMR: Major (Z) isomer R_{f} 0.30 (cyclohexane/AcOEt 8:2) 5.79 (*dt*, *J* = 1.5, 6.7, 1H); 3.74 (*dd*, *J* = 5.7, 10.5, 1H); 3.39 (*dd*, *J* = 8.5, 10.5, 1H); 2.18-2.11 (*m,* 2H); 2.02 (br*s*, 1 OH); 1.79-1.74 (*m,* 1H); 1.43-1.37 (*m,* 2H); 1.38-1.23 (*m,* 10H); 0.88 (*t*, *J* = 7, 3H). Minor (E) isomer R_{f} = 0.27 (cyclohexane/AcOEt 8 :2) 5.85 (*qq*, *J* = 1.9, 6.6, 1H); 3.58 (*dd, J* = 6.5, 11.2, 1H); 3.48 (*dd*, *J* = 7.4, 11.2, 1H); 2.165 (*q*, *J* = 7, 2H); 2.00 (br*s*, 1 OH); 1.78-1.73 (*m,* 1H); 1.45-1.39 (*m,* 2H); 1.34-1.24 (*m,* 10H); 0.88 (*t, J* = 7, 3H).

¹³C-NMR: Major (Z) isomer R_{f} 0.30 (cyclohexane/AcOEt 8:2) 122.7 (*s*); 120.4 (*d*); 65.7 (*t*); 32.3 (*t*); 31.9 (*t*); 29.6 (*t*); 29.2 (2*t*); 22.7 (*t*); 17.8 (*d*); 14.1 (*q*); 7.7 (*t*). Minor (E) isomer R_{f} = 0.27 (cyclohexane/AcOEt 8:2) 122.9 (*s*); 119.8 (*d*); 65.9 (*t*); 31.9 (*t*); 31.7 (*t*); 29.2 (3*t*); 22.7 (*t*); 17.6 (*d*); 14.1 (*q*); 7.3 (*t*).

### d) Step 4; preparation of 2-octylcyclopropyl-1-methanol

Usual hydrogenation of crude (2-octylidenecyclopropyl)methanol (as a 66:33 *(Z)*/*(E)* mixture) over *Lindlar* catalyst afforded quantitatively a 60:40 *cis*/*trans* mixture of 2-octylcyclopropyl-1-methanol.

Analyses corresponding to those reported in Example 5, Step 4 for 2-octylcyclopropyl-1-methanol.

### e) Step 5: preparation of the comparative composition of matter

Usual *Jones* oxidation of a 60:40 *cis*/*trans* mixture of 2-octylcyclopropyl-1-methanol as above, followed by CC/SiO₂ (cyclohexane/AcOEt 8:2) purification and bulb-to-bulb distillation afforded a 52:48 mixture of cis-(1SR,2RS)-2-octylcyclopropanecarboxylic acid / trans-(1RS,2RS)-2-octylcyclopropanecarboxylic acid.

Analyses in accord with those of the optically active stereoisomers, as described in Example 3 and 8.

### Example 7

### Synthesis of a comparative composition of matter

### a) Step 1: preparation of ethyl 2-octylcyclopropane-1-carboxylate

A soln. of ethyldiazoacetate (85%/CH₂Cl₂, 7.5g, 55.9 mmol) in 1-decene (10.0 g, 71.4 mmol) was added dropwise in 6h to a suspension of anh. CuSO₄ (2.0 g, 12.5 mmol) in 1-decene (9.0 g, 64.3 mmol) at 100°C. The addition is accompanied by N₂ evolution. After completion, the temperature was maintained until evolution stopped. CuSO₄ was filtered, and the reaction mixture was purified by CC/SiO₂ (cyclohexane/AcOEt 99:1 to 9:1). Unreacted 1-decene was recuperated, as well as ester ethyl cis-2-octylcyclopropane-1-carboxylate in 42% yield, (80% yield based on used/recuperated) as a 40:60 cis/trans mixture of stereoisomer.

Analyses of the cis-diastereoisomer are reported in Example 2. ¹³C-NMR: *trans* isomer tentatively deduced from the mixture: 174.7 (*s*); 60.3 (*t*); 33.1 (*t*) 29.6 (*t*); 29.4 (*t*); 29.3 (*t*); 29.1 (*t*); 23.0 (*d*); 22.7 (*t*); 20.3 (*d*); 15.6 (*t*); 14.3 (*q*); 14.1 (*q*); 13.2 (*t*).

### b) Step 2: preparation of comparative composition of matter

A 40:60 cis/trans mixture of ethyl cis-2-octylcyclopropane-1-carboxylate (3130 mg, 13.83 mmol), and LiOH (828 mg, 34.6 mmol) in THF (10 ml) and H₂O (2.5 ml) was heated at reflux for 56h. The cold reaction mixture was partitioned between Et₂O (4 x 25 ml) and H₂O (4 x 25 ml). The aq. Phase was acidified to pH 1 with 37% HCl, then re-extracted with Et₂O (4 x 25 ml), and washed with H₂O (2 x 25 ml), dried (Na₂SO₄), then concentrated to afford a 96% pure 40:60 mixture of cis-(1SR,2RS)-2-octylcyclopropanecarboxylic acid / trans-(1SR,2SR)-2-octylcyclopropanecarboxylic acid in 90% yield. Analyses in accord with those of the optically active stereoisomers, as described in Example 3 and in Example 8.

### Example 8

### Synthesis of a comparative compound (+)-trans-(1S,2S)-2-octylcyclopropanecarboxylic acid

Obtained from the known undec-2-yn-1-ol [Synthesis 1999, 107] according to the procedure published *in* Angew. Chem. Int. Ed. 2016, 55, 13719. Then purification by CC/SiO₂ (cyclohexane/AcOEt 99/1 to 9/1) to reach a chemical purity of 98.9%. R_{f} = 0.42 (c-hexane/AcOEt 7:3). [α]_{D}²⁰ = +63.1 (c = 0.045, CHCl₃). ¹H-NMR: 11.46 (br*s*, 1 OH); 1.45-1.37 (*m,* 4H); 1.34 (*dt*, *J* = 4, 8, 2H); 1.32-1.24 (*m,* 8H); 1.22 (*dt*, *J* = 4.3, 8.6, 2H); 0.88 (*t*, *J* = 7, 3H); 0.77 (*dt*, *J* = 4.2, 7.2, 2H). ¹³C-NMR: 181.3 (*s*); 33.1 (*t*); 31.9 (*t*); 29.5 (*t*); 29.3 (2*t*); 29.1 (*t*); 24.1 (*d*); 22.7 (*t*); 20.1 (*d*); 16.4 (*t*) ; 14.1 (*q*).

### Example 9

### Synthesis of a comparative composition of matter

Obtained from the known undec-2-yn-1-ol [Synthesis 1999, 107] according to the procedure published *in* Angew. Chem. Int. Ed. 2016, 55, 13719. Then purification by CC/SiO₂ (cyclohexane/AcOEt 99/1 to 9/1) to reach a stereochemical purity of 94% (-)-cis-(1R,2S)-2-octylcyclopropanecarboxylic acid and 6% (+)-trans-(1S,2S)-2-octylcyclopropanecarboxylic acid. Bp: 175°C/3 mbar. [α]_{D}²⁰ = -29.0 (c = 0.039, CHCl₃). For analyses, see Example 3.

### Example 10

### Preparation of a perfuming composition

A perfuming composition for fine fragrance was prepared by admixing the following ingredients:

| **Ingredient Name** | **Parts by weight** |
|---|---|
| (2E)-2-benzylideneoctanal | 100 |
| Mixture of (E)-2-dodecenaL, (Z)-3-dodecenal and dodecanal | 10 |
| (-)-(3aR,5aS,9aS,9bR)-3a,6,6,9a-tetramethyldodecahydronaphtho[2,1-b]furan | 200 |
| 10% * 1-(5-propyl-1,3-benzodioxol-2-yl)ethanone | 20 |
| Cassis base¹⁾ | 100 |
| (3Z)-3-hexen-1-ol | 20 |
| 3,7-dimethyl-6-octen-1-ol | 100 |
| Cypres oil | 20 |
| Estragon oil | 20 |
| Oxacyclohexadecan-2-one | 400 |
| (E)-3,7-dimethyl-2,6-octadien-1-ol | 100 |
| 1-oxa-12/13-cyclohexadecen-2-one | 400 |
| Methyl 2-((1RS,2RS)-3-oxo-2-pentylcyclopentyl)acetate | 1600 |
| Mixture of methyl [(1RS,2RS)-3-oxo-2-pentylcyclopentyl]acetate and methyl [(1RS,2SR)-3-oxo-2-pentylcyclopentyl]acetate | 1000 |
| 3-(1,3-benzodioxol-5-yl)-2-methylpropanal | 200 |
| 2-{(1S)-1-[(1R)-3,3-dimethylcyclohexyl]ethoxy}-2-methylpropyl propionate | 800 |
| 7-hydroxy-3,7-dimethyloctanal | 200 |
| Mixture of (3E)-3-methyl-4-(2,6,6-trimethyl-2-cyclohexen-1-yl)-3-buten-2-one and (1E)-1-(2,6,6-trimethyl-2-cyclohexen-1-yl)-1-penten-3-one | 40 |
| 6,6-dimethoxy-2,5,5-trimethyl-2-hexene | 100 |
| 4-(2,2,C-3,T-6-tetramethyl-R-1-cyclohexyl)-3-buten-2-one | 20 |
| Orange oil | 1000 |
| (1S,1'R)-[1-(3',3'-Dimethyl-1'-cyclohexyl)ethoxycarbonyl]methyl propanoate | 1600 |
| (-)-(2S)-1-oxo-1-(2-propanyloxy)-2-propanyl 2,2-dimethylpropanoate | 400 |
| (2Z)-2-phenyl-2-hexenenitrile | 20 |
| (-)-propyl (2S)-2-[(2-methyl-2-butanyl)oxy]propanoate | 200 |
| Mixture of 1-(1,2,3,4,5,6,7,8-octahydro-2,3,8,8-tétramethyl-2-naphtyl)ethan-1-one and isomers | 1000 |
| 2-(2-methyl-2-propanyl)cyclohexyl acetate | 80 |
| (3E)-4-(2,6,6-trimethyl-1-cyclohexen-1-yl)-3-buten-2-one | 40 |
| 2,4-dimethyl-3-cyclohexene-1-carbaldehyde | 10 |
| | **9800** |

| | |
|---|---|
| * in dipropyleneglycol 1) Origin: Firmenich SA, Geneva, Switzerland | |

The addition of 200 parts by weight of the invention's composition of matter described in Example 1 diluted at 10% in dipropyleneglycol to the above-described fine fragrance composition imparted to the latter an intense and natural incense effect which brings a lot of strength and diffusion to the fragrance. The invention's composition of matter blends particularly well with citrus elements such as 6,6-dimethoxy-2,5,5-trimethyl-2-hexene, (E)-2-dodecenaL, (Z)-3-dodecenal and dodecanal, woody-powdery elements such as 4-(2,2,C-3,T-6-tetramethyl-R-1-cyclohexyl)-3-buten-2-one and mixture of 1-(1,2,3,4,5,6,7,8-octahydro-2,3,8,8-tetramethyl-2-naphtyl)ethan-1-one and isomers and amber elements, in particular (-)-(3aR,5aS,9aS,9bR)-3a,6,6,9a-tetramethyldodecahydronaphtho[2,1-b]furan.

The addition of the same amount of 200 parts by weight of the comparative composition of matter described in Example 5 diluted at 10% in dipropyleneglycol imparted an incense and citrus character but allied with fatty and dusty notes. The fine fragrance composition obtained by this addition is devoid of clean effect.

The addition of the same amount of 200 parts by weight of the comparative composition of matter described in Example 6 diluted at 10% in dipropyleneglycol imparted a similar effect than with the addition of comparative composition of matter described in Example 5 with stronger undesired fatty and dusty notes. Said composition was also less powerful than the invention's composition.

The addition of the same amount of 200 parts by weight of the comparative composition of matter described in Example 7 diluted at 10% in dipropyleneglycol imparted mainly an unpleasant fatty and dusty characters. Said composition was also less powerful than the invention's composition.

The addition of the same amount of 200 parts by weight of the comparative composition of matter described in Example 8 diluted at 10% in dipropyleneglycol imparted mainly an unpleasant fatty character. Said composition was also less powerful than the invention's composition.

### Example 11

### Preparation of a eau de toilette comprising the invention's composition of matter

The eau de toilette was prepared by adding 15% by weight, relative to the total weight of the eau de toilette, of the invention's composition of example 10 into ethanol.

### Example 12

### Preparation of a liquid detergent comprising the invention's compound

**Table 1: Composition of the liquid detergent formulation**

| **Ingredients** | **Concentration [wt%]** |
|---|---|
| Sodium C14-17 Alkyl Sec Sulfonate¹⁾ | 7 |
| Fatty acids, C12-18 and C18-unsaturated²⁾ | 7.5 |
| C12/14 fatty alcohol polyglycol ether with 7 mol EO³⁾ | 17 |
| Triethanolamine | 7.5 |
| Propylene Glycol | 11 |
| Citric acid | 6.5 |
| Potassium Hydroxyde | 9.5 |
| Properase L⁴⁾ | 0.2 |
| Puradax EG L⁴⁾ | 0.2 |
| Purastar ST L⁴⁾ | 0.2 |
| Acrylates/Steareth-20 Methacrylate structuring Crosspolymer⁵⁾ | 6 |
| Deionized Water | 27.4 |

| | |
|---|---|
| 1) Hostapur SAS 60; Origin: Clariant 2) Edenor K 12-18; Origin: Cognis 3) Genapol LA 070; Origin: Clariant 4) Origin: Genencor International 5) Aculyn 88; Origin: Dow Chemical | |

The liquid detergent is prepared by adding 0.5 to 1.5 % by weight, relative to the total weight of the liquid detergent, of the invention's composition of Example 10 comprising 200 parts by weight of the invention's composition of matter described in Example 1 diluted at 10% in dipropyleneglycol into the unperfumed liquid detergent formulation of Table 1 under gentle shaking.

### Example 13

### Preparation of a fabric softener comprising the invention's compound

**Table 2: Composition of the softener formulation**

| **Ingredient** | **Concentration [wt%]** |
|---|---|
| Methyl bis[ethyl (tallowate)]-2-hydroxyethyl ammonium methyl sulfate¹⁾ | 12.20 |
| 1,2-benzisothiazolin-3-one²⁾ | 0.04 |
| CaCl₂ (10% aqueous solution) | 0.40 |
| Water | 87.36 |

| | |
|---|---|
| 1) Stepantex VL90 A Diester Quat; Origin: Stepan 2) Proxel GXL; Origin: Arch | |

The softener is prepared by weighting Methyl bis[ethyl (tallowate)]-2- hydroxyethyl ammonium methyl sulfate which was heated at 65°C. Then Water and 1,2-benzisothiazolin-3-one are placed in the reactor and are heated at 65°C under stirring. To the above mixture is added Methyl bis[ethyl (tallowate)]-2- hydroxyethyl ammonium methyl sulfate. The mixture is stirred 15 minutes and CaCl₂ is added. Then 0.5 to 2% by weight, relative to the total weight of the softener, of the invention's composition of Example 10 comprising 200 parts by weight of the invention's composition of matter described in Example 1 diluted at 10% in dipropyleneglycol is added. The mixture is stirred 15 minutes and is cooled down to room temperature under stirring (viscosity measure : result 35 +/- 5 mPas. (shear rate 106 sec-1)).

### Example 14

### Preparation of a transparent isotropic shampoo comprising the invention's composition

**Table 3: Composition of the transparent isotropic shampoo formulation**

| **Phases** | **Ingredients** | **Concentration [wt%]** |
|---|---|---|
| **A** | Water deionized | 44.4 |
| | Polyquaternium-10 ¹⁾ | 0.3 |
| | Glycerin 85% ²⁾ | 1 |
| | DMDM Hydantoin ³⁾ | 0.2 |
| **B** | Sodium Laureth Sulfate ⁴⁾ | 28 |
| | Cocamidopropyl Betaine ⁵⁾ | 3.2 |
| | Disodium Cocoamphodiacetate ⁶⁾ | 4 |
| | Ethoxy (20) Stearyl Alcohol ⁶⁾ | 1 |
| **C** | Sodium Laureth Sulfate ⁴⁾ | 3 |
| | Glyceryl Laureate ⁷⁾ | 0.2 |
| **D** | Water deionized | 1 |
| | Sodium Methylparaben ⁸⁾ | 0.1 |
| **E** | Sodium Chloride 10% aqueous sol. | 15 |
| | Citric acid 10% aqueous sol. till pH 5.5-6 | q.s. |

| | | |
|---|---|---|
| 1) Ucare Polymer JR-400, Origin: Noveon 2) Origin: Schweizerhall 3) Glydant, Origin: Lonza 4) Texapon NSO IS, Origin: Cognis 5) Tego Betain F 50, Origin: Evonik 6) Amphotensid GB 2009, Origin: Zschimmer & Schwarz 7) Monomuls 90 L-12, Origin: Gruenau 8) Nipagin Monosodium, Origin: NIPA | | |

The shampoo is prepared by dispersed in water Polyquaternium-10. The remaining ingredients of phase A are mixed separately by addition of one after the other while mixing well after each adjunction. This pre-mix is added to the Polyquaternium-10 dispersion and mixed for another 5 min. Then, the premixed phase B and the premixed Phase C are added (Monomuls 90L-12 is heated to melt in Texapon NSO IS) while agitating. Phase D and Phase E are added while agitating. PH is adjusted with citric acid solution till pH: 5.5 - 6.0 leading to an unperfumed shampoo formula.

The perfumed shampoo is prepared by adding 0.4 to 0.8% by weight, relative to the total weight of the shampoo, of the invention's composition of Example 10 comprising 200 parts by weight of the invention's composition of matter described in Example 1 diluted at 10% in dipropyleneglycol into the unperfumed shampoo formulation of Table 3 under gentle shaking.

### Example 15

### Preparation of a structured shower gel comprising the invention's composition

**Table 4: Composition of the shower gel formulation**

| **Ingredients** | **Amount (% wt)** |
|---|---|
| WATER deionised | 49.350 |
| Tetrasodium EDTA ¹⁾ | 0.050 |
| Acrylates Copolymer²⁾ | 6.000 |
| Sodium C12-C15 Pareth Sulfate ³⁾ | 35.000 |
| Sodium Hydroxide 20% aqueous solution | 1.000 |
| Cocamidopropyl Betaine⁴⁾ | 8.000 |
| Methylchloroisothiazolinone and Methylisothiazolinone⁵⁾ | 0.100 |
| Citric Acid (40%) | 0.500 |

| | |
|---|---|
| 1) EDETA B POWDER; trademark and origin: BASF 2) CARBOPOL AQUA SF-1 POLYMER; trademark and origin: NOVEON 3) ZETESOL AO 328 U; trademark and origin: ZSCHIMMER & SCHWARZ 4) TEGO-BETAIN F 50; trademark and origin: GOLDSCHMIDT 5) KATHON CG; trademark and origin: ROHM & HASS | |

The shower gel is prepared by adding 0.5 to 1.5% by weight, relative to the total weight of the shower gel, of the invention's composition of Example 10 comprising 200 parts by weight of the invention's composition of matter described in Example 1 diluted at 10% in dipropyleneglycol into the unperfumed shower gel formulation of Table 4 under gentle shaking.

### Example 16

### Preparation of a transparent shower gel comprising the invention's composition

**Table 5: Composition of the transparent shower gel formulation**

| **Ingredients** | **Concentration (% wt)** |
|---|---|
| WATER deionized | 52.40 |
| Tetrasodium EDTA ¹⁾ | 0.10 |
| Sodium Benzoate | 0.50 |
| Propylene Glycol | 2.00 |
| Sodium C12-C15 Pareth Sulfate ²⁾ | 35.00 |
| Cocamidopropyl Betaine³⁾ | 8.00 |
| Polyquaternium-7⁴⁾ | 0.20 |
| Citric Acid (40%) | 1.00 |
| Sodium Chloride | 0.80 |

| | |
|---|---|
| 1) EDETA B POWDER; trademark and origin: BASF 2) ZETESOL AO 328 U; trademark and origin: ZSCHIMMER & SCHWARZ 3) TEGO-BETAIN F 50; trademark and origin: GOLDSCHMIDT 4) MERQUAT 550; trademark and origin: LUBRIZOL | |

The transparent shower gel is prepared by adding 0.5 to 1.5% by weight, relative to the total weight of the shower gel, of the invention's composition of Example 10 comprising 200 parts by weight of the invention's composition of matter described in Example 1 diluted at 10% in dipropyleneglycol into the unperfumed shower gel formulation of Table 5 under gentle shaking.

### Example 17

### Preparation of a milky shower gel comprising the invention's composition

**Table 6: Composition of the milky shower gel formulation**

| **Ingredients** | **Concentration (% wt)** |
|---|---|
| WATER deionized | 50.950 |
| Tetrasodium EDTA ¹⁾ | 0.050 |
| Sodium Benzoate | 0.500 |
| Glycerin 86% | 3.500 |
| Sodium Laureth Sulfate ²⁾ | 27.000 |
| Polyquaternium-7³⁾ | 1.000 |
| Coco-Betaine⁴⁾ | 6.000 |
| PEG-120 Methyl Glucose trioleate⁵⁾ | 1.000 |
| Citric Acid (40%) | 1.000 |
| Glycol Distearate & Laureth-4 & Cocamidopropyl Betaine⁶⁾ | 3.000 |
| Sodium Chloride 20% | 5.000 |
| PEG-40 Hydrogenated Castor Oil⁷⁾ | 1.000 |

| | |
|---|---|
| 1) EDETA B POWDER; trademark and origin: BASF 2) Texapon NSO IS; trademark and origin: COGNIS 3) MERQUAT 550; trademark and origin: LUBRIZOL 4) DEHYTON AB-30; trademark and origin: COGNIS 5) GLUCAMATE LT; trademark and origin: LUBRIZOL 6) EUPERLAN PK 3000 AM; trademark and origin: COGNIS 7) CREMOPHOR RH 40; trademark and origin: BASF | |

The transparent shower gel is prepared by adding 0.5 to 1.5% by weight, relative to the total weight of the shower gel, of the invention's composition of Example 10 comprising 200 parts by weight of the invention's composition of matter described in Example 1 diluted at 10% in dipropyleneglycol into the unperfumed shower gel formulation of Table 6 under gentle shaking.

### Example 18

### Preparation of a pearly shampoo comprising the invention's composition

**Table 7: Composition of the pearly isotropic_shampoo formulation**

| **Phases** | **Ingredients** | **Concentration (% wt)** |
|---|---|---|
| **A** | Water deionized | 45.97 |
| | Tetrasodium EDTA ¹⁾ | 0.05 |
| | Guar Hydroxypropyltrimonium Chloride ²⁾ | 0.05 |
| | Polyquaternium-10 ³⁾ | 0.075 |
| **B** | NaOH 10% aqueous sol. | 0.3 |
| **C** | Ammonium Lauryl Sulfate ⁴⁾ | 34 |
| | Ammonium Laureth Sulfate ⁵⁾ | 9.25 |
| | Cocamidopropyl Betaine ⁶⁾ | 2 |
| | Dimethicone (&) C12-13 Pareth-4 (&) C12-13 Pareth-23 (&) Salicylic Acid ⁷⁾ | 2.5 |
| **D** | Cetyl Alcohol ⁸⁾ | 1.2 |
| | Cocamide MEA ⁹⁾ | 1.5 |
| | Glycol Distearate ¹⁰⁾ | 2 |
| **E** | Methylchloroisothiazolinone & Methylisothiazolinone 11) | 0.1 |
| | D-Panthenol 75% ¹²⁾ | 0.1 |
| | Water deionized | 0.3 |
| **F** | Sodium Chloride 25% aqueous sol. | 0.6 |

| | | |
|---|---|---|
| 1) EDETA B Powder, Origin: BASF 2) Jaguar C14 S, Origin: Rhodia 3) Ucare Polymer JR-400, Origin: Noveon 4) Sulfetal LA B-E, Origin: Zschimmer & Schwarz 5) Zetesol LA, Origin: Zschimmer & Schwarz 6) Tego Betain F 50, Origin: Evonik 7) Xiameter MEM-1691, Origin: Dow Corning 8) Lanette 16, Origin: BASF 9) Comperlan 100, Origin: Cognis 10) Cutina AGS, Origin: Cognis 11) Kathon CG, Origin: Rohm & Haas 12) D-Panthenol, Origin: Roche | | |

The shampoo is prepared by dispersed in water and Tetrasodium EDTA, Guar Hydroxypropyltrimonium Chloride and Polyquaternium-10. NaOH 10% solution (Phase B) is added once Phase A is homogeneous. Then, the premixed Phase C is added. and mixture is heated to 75°C. Phase D ingredients are added and mixed till homogeneous. The mixture is cooled down. At 45°C, Phase E ingredients are added while mixing. Final viscosity is adjusted with 25% NaCl solution and pH of 5.5-6 is adjusted with 10% NaOH solution.

The perfumed pearly shampoo is prepared by adding 0.4 to 0.8% by weight, relative to the total weight of the shampoo, of the invention's composition of Example 10 comprising 200 parts by weight of the invention's composition of matter described in Example 1 diluted at 10% in dipropyleneglycol into the unperfumed shampoo formulation of Table 7 under gentle shaking.

### Example 19

### Preparation of a structured shower gel comprising the invention's composition

**Table 8: Composition of the milky shower gel formulation**

| **Ingredients** | **Amount (% wt)** |
|---|---|
| WATER deionised | 49.350 |
| Tetrasodium EDTA ¹⁾ | 0.050 |
| Acrylates Copolymer²⁾ | 6.000 |
| Sodium C12-C15 Pareth Sulfate ³⁾ | 35.000 |
| Sodium Hydroxide 20% aqueous solution | 1.000 |
| Cocamidopropyl Betaine⁴⁾ | 8.000 |
| Methylchloroisothiazolinone and Methylisothiazolinone⁵⁾ | 0.100 |
| Citric Acid (40%) | 0.500 |

| | |
|---|---|
| 6) EDETA B POWDER; trademark and origin: BASF 7) CARBOPOL AQUA SF-1 POLYMER; trademark and origin: NOVEON 8) ZETESOL AO 328 U; trademark and origin: ZSCHIMMER & SCHWARZ 9) TEGO-BETAIN F 50; trademark and origin: GOLDSCHMIDT 10) KATHON CG; tradeark and origin: ROHM & HASS | |

The transparent shower gel is prepared by adding 0.5 to 1.5% by weight, relative to the total weight of the shower gel, of the invention's composition of Example 10 comprising 200 parts by weight of the invention's composition of matter described in Example 1 diluted at 10% in dipropyleneglycol into the unperfumed shower gel formulation of Table 8 under gentle shaking.

### Example 20

### Preparation of anhydrous antiperspirant spray formulations comprising the invention's composition

**Table 9: Composition of the anhydrous antiperspirant spray formulation**

| **Ingredients** | **Amount (% wt)** |
|---|---|
| Cyclomethicone ⁽¹⁾ | 53.0 |
| Isopropyl myristate | 9.0 |
| Silica ⁽²⁾ | 1.0 |
| Quaternium-18-hectorite ⁽³⁾ | 3.3 |
| Aluminium chlorohydrate ⁽⁴⁾ | 32.7 |
| Perfume oil | 1 |

| | |
|---|---|
| ⁽¹⁾ Dow Corning^{®} 345 Fluid; origin: Dow Corning ⁽²⁾ Aerosil^{®} 200 ; origin: Evonik ⁽³⁾ Bentone^{®} 38; origin: Elementis Specialities ⁽⁴⁾ Micro Dry Ultrafine; origin: Reheis | |

Anhydrous antiperspirant spray formulation is prepared by using a high speed stirrer. Silica and Quaternium-18-hectorite are added to the mixture of isopropyl myristate and cyclomethicone. Once completely swollen, aluminium chlorohydrate is added portion-wise under stirring until the mixture becomes homogeneous and without lumps. Then a perfume oil being the invention's composition of Example 10 comprising 200 parts by weight of the invention's composition of matter described in Example 1 diluted at 10% in dipropyleneglycol is added.

### Example 21

### Preparation of deodorant spray emulsion formulations comprising the invention's composition

**Table 10: Composition of deodorant spray emulsion formulation**

| **Ingredients** | **Amount (% wt)** |
|---|---|
| Ethanol (95%) | 89.25 |
| Triclosan ⁽¹⁾ | 0.25 |
| Isopropyl myristate | 9.00 |
| Invention's composition of Example 10 comprising 200 parts by weight of the invention's composition of matter described in Example 1 diluted at 10% in dipropyleneglycol | 1.5 |

| | |
|---|---|
| ⁽¹⁾ Irgasan^{®} DP 300; origin: BASF ⁽²⁾ mixture of Compounds 2a/2b ca. 45:55 | |

Deodorant spray emulsion formulation is prepared by mixing and dissolving all the ingredients according to the sequence of Table 10. Aerosol cans are filled, and the propellant is crimped and added. Aerosol filling: 40% active solution 60% propane / butane (2.5 bar).

### Example 22

### Preparation of deodorant stick formulations comprising the invention's composition

**Table 11: Composition of Deodorant stick formulation**

| **Phase** | **Ingredients** | **Amount (% wt)** |
|---|---|---|
| A | Stearic acid | 5.00 |
| | 1,2-Propylene glycol | 41.45 |
| | Sodium hydroxide (20% aqueous solution) | 4.20 |
| | Water | 30.00 |
| | Tetrasodium EDTA ⁽¹⁾ | 0.10 |
| | Ceteareth-25 ⁽²⁾ | 1.50 |
| | PPG-3 Myristyl ether ⁽³⁾ | 1.50 |
| B | 1,2-Propylene glycol | 15.00 |
| | Triclosan ⁽⁴⁾ | 0.25 |
| C | Perfume oil | 1 |

| | | |
|---|---|---|
| ⁽¹⁾ Edeta^{®} B Power; origin: BASF ⁽²⁾ Cremophor^{®} A25; origin: BASF ⁽³⁾ Tegosoft^{®} APM; origin: Evonik ⁽⁴⁾ Irgasan^{®} DP 300; origin: BASF | | |

Deodorant stick formulation is btained by weighing all the components of Part A and heating to 70-75°C. Ceteareth-25 is added once the other Part A ingredients are mixed and heated. When the Ceteareth-25 is dissolved, stearic acid is added. Part B is prepared by dissolving Triclosan in 1,2-propylene glycol. Evaporated water is compensated. Then, slowly, under mixing, Part B is poured into Part A. A perfume oil being the invention's composition of Example 10 comprising 200 parts by weight of the invention's composition of matter described in Example 1 diluted at 10% in dipropyleneglycol (Phase C) is added under gentle shaking. To stock, a plastic bag is put into the bucket to be sealed after cooling. Moulds were filled at about 70°C.

### Example 23

### Preparation of deodorant roll-on formulations comprising the invention's composition

**Table 12: Composition of deodorant roll-on formulation**

| **Phase** | **Ingredients** | **Amount (% wt)** |
|---|---|---|
| A | Water | 50.00 |
| | Hydroxyethylcellulose ⁽¹⁾ | 0.70 |
| B | Ethanol (95%) | 40.00 |
| | 1,2-Propylene glycol | 5.00 |
| | Triclosan ⁽²⁾ | 0.30 |
| C | PEG-40 hydrogenated castor oil ⁽³⁾ | 3.00 |
| D | Invention's composition of Example 10 comprising 200 parts by weight of the invention's composition of matter described in Example 1 diluted at 10% in dipropyleneglycol | 1 |

| | | |
|---|---|---|
| ⁽¹⁾ Natrosol^{®} 250 H; origin: Ashland ⁽²⁾ Irgasan^{®} DP 300; origin: BASF ⁽³⁾ Cremophor^{®} RH 40; origin: BASF | | |

Part A is prepared by sprinkling little-by-little the hydroxyethylcellulose into the water, whilst rapidly stirring with a turbine until the hydroxyethylcellulose is entirely swollen giving a limpid gel. Part B is slowly poured into Part A, whilst continuing stirring until the entire mixture is homogeneous. Then Parts C and D are added under gentle shaking.

### Example 24

### Preparation of day cream base O/W emulsions comprising the invention's composition

**Table 13: Composition of day cream base O/W emulsion formulation**

| **Phase** | **Ingredients** | **Amount (% wt)** |
|---|---|---|
| A | Steareth-2 (and) PEG-8 Distearate⁽¹⁾ | 5.0 |
| | Cetyl alcohol | 0.5 |
| | Ceteth-20 (AND) glyceryl stearate (and) PEG-6 stearate (and) Steareth-20 ⁽²⁾ | 4.0 |
| | Squalan ⁽³⁾ | 1.0 |
| | Paraffin oil ⁽⁴⁾ | 2.0 |
| | Petrolatum ⁽⁵⁾ | 5.5 |
| B | Deionized water | 75.9 |
| | Propylene glycol | 5.0 |
| C | Phenoxyethanol (AND) Piroctone olamine ⁽⁶⁾ | 0.6 |
| D | Sodium carbomer ⁽⁷⁾ | 0.2 |
| E | Perfume oil | 0.3 |

| | | |
|---|---|---|
| ⁽¹⁾ Arlacel^{®} 985; origin: Croda ⁽²⁾ Tefose^{®} 2561; origin: Gattefossé ⁽³⁾ Biolip P 90; origin: Gattefossé ⁽⁴⁾ Mineral oil 30-40 CPS ⁽⁵⁾ Petroleum jelly ⁽⁶⁾ Nipaguard^{®} PO 5; origin: Clariant ⁽⁷⁾ PNC 400 | | |

Day cream base O/W emulsions is prepared by heating Phases A and B separately to 70-75 °C. Phase A is added to Phase B, then vacuum is applied. The mixture is stirred and cooled to 55°C for 15 min. After cooling to room temperature, phenoxyethanol (and) piroctone olamine (Part C) are added when a temperature of 45°C is reached. The mixture is stirred for 5 min before sodium carbomer (Part D) and a perfume oil being the invention's composition of Example 10 comprising 200 parts by weight of the invention's composition of matter described in Example 1 diluted at 10% in dipropyleneglycol (Part E) is added. The mixture is stirred for 3 min, then the stirring is stopped for 15 min. When the temperature of the mixture reaches 30°C, the stirring is resumed for another 15 min until the cream becomes homogeneous, glossy and without lumps. If necessary the pH is adjusted to 6.70-7.20 with Glydant, Phenonip or Nipaguard POS or to 6.30-7.00 with Nikkoguard.

## Claims

1. A composition of matter comprising a compound of formula (I) 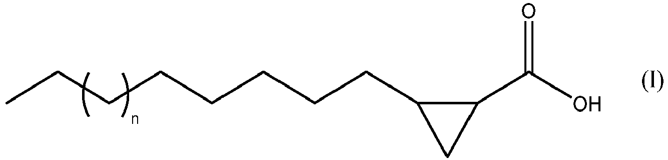 wherein n is 1 or 2; the weight ratio of the cis-diastereoisomers to the trans-diastereoisomers is comprised in the range between 90:10 and 99.5:0.5 and comprising at least 10% w/w of cis-(1S,2R) diastereoisomer, the percentage being relative to the total weight of the composition of matter.

2. The composition of matter according to claim 1, wherein the composition of matter comprises:
a) from 90 to 99.5% w/w of cis-2-octylcyclopropanecarboxylic acid;
b) from 0.5% to 10% w/w of trans-2-octylcyclopropanecarboxylic acid;
the percentage being relative to the total weight of the composition of matter.

3. The composition of matter according to any one of claims 1 to 2, wherein the composition of matter comprises at least 45% w/w of cis-(1S,2R)-2-octylcyclopropanecarboxylic acid, the percentage being relative to the total weight of the composition of matter.

4. The composition of matter according to any one of claims 1 to 3, wherein the composition of matter comprises:
a) from 45 to 99.5% w/w of cis-(1S,2R)-2-octylcyclopropanecarboxylic acid;
b) at most 49,75% w/w of cis-(1R,2S)-2-octylcyclopropanecarboxylic acid;
c) at most 10% w/w of trans-(1S,2S)-2-octylcyclopropanecarboxylic acid;
d) at most 10% w/w of trans-(1R,2R)-2-octylcyclopropanecarboxylic acid;
the percentage being relative to the total weight of the composition of matter; and
wherein the weight ratio of the cis-diastereoisomers to the trans-diastereoisomers is comprised in the range between 90:10 and 99.5:0.5.

5. The composition of matter according to any one of claims 1 to 4, wherein the composition of matter comprises:
a) from 47.5 to 99% w/w of cis-(1S,2R)-2-octylcyclopropanecarboxylic acid;
b) at most 49.5% w/w of cis-(1R,2S)-2-octylcyclopropanecarboxylic acid;
c) at most 5% w/w of trans-(1S,2S)-2-octylcyclopropanecarboxylic acid;
d) at most 5% w/w of trans-(1R,2R)-2-octylcyclopropanecarboxylic acid.

6. The composition of matter according to any one of claims 1 to 5, wherein the weight ratio of the cis-diastereoisomers to the trans-diastereoisomers is comprised in the range between 95:5 and 99:1.

7. The composition of matter according to any one of claims 1 to 6, wherein cis-2-octylcyclopropanecarboxylic acid and trans-2-octylcyclopropanecarboxylic acid are in a racemic form.

8. The composition of matter according to any one of claims 1 to 7, wherein the composition of matter comprises:
a) from 47.5 to 49.75% w/w of cis-(1S,2R)-2-octylcyclopropanecarboxylic acid;
b) from 47.5 to 49.75% w/w of cis-(1R,2S)-2-octylcyclopropanecarboxylic acid;
c) from 0.5% to 2.5% w/w of trans-(1S,2S)-2-octylcyclopropanecarboxylic acid;
d) from 0.5% to 2.5% w/w of trans-(1R,2R)-2-octylcyclopropanecarboxylic acid;

9. A method to confer, enhance, improve or modify the odor properties of a perfuming composition or of a perfumed article or of a surface, which method comprises adding to said composition or perfumed article an effective amount of a composition of matter as defined in any one of claims 1 to 8.

10. Use as perfuming ingredient of a composition of matter as defined in any one of claims 1 to 8.

11. A perfuming composition comprising
i) a composition of matter, as defined in any one of claims 1 to 8;
ii) at least one ingredient selected from the group consisting of a perfumery carrier and a perfumery base; and
iii) optionally at least one perfumery adjuvant.

12. A perfumed consumer product comprising a composition of matter as defined in any one of claims 1 to 8 or a perfuming composition as defined in claim 11.

13. The perfumed consumer product according to claim 12, wherein the perfumery consumer product is a perfume, a fabric care product, a body-care product, a cosmetic preparation, a skin-care product, an air care product or a home care product.

14. The perfumed consumer product according to claim 13, wherein the perfumery consumer product is a fine perfume, a splash or eau de parfum, a cologne, a shave or after-shave lotion, a liquid, a pod, or solid detergent or tablet, a fabric softener, a liquid or solid scent booster, a dryer sheet, a fabric refresher, an ironing water, a paper, a bleach, a carpet cleaner, a curtain-care product, a shampoo, a leave-on or rinse-off hair conditioner, a coloring preparation, a color-care product, a hair shaping product, a dental care product, a disinfectant, an intimate care product, a hair spray, a skin cream or lotion, a vanishing cream, a deodorant or antiperspirant, a hair remover, a tanning or sun or after sun product, a nail product, a skin cleansing, a makeup, a perfumed soap, a shower or bath mousse, oil or gel, a foot/hand care product, a hygiene product, an air freshener, a "ready to use" powdered air freshener, a mold remover, a furnisher care, a wipe, a dish detergent or hard-surface detergent, a leather care product, a car care product.

## Patentansprüche

1. Stoffzusammensetzung, umfassend eine Verbindung der Formel (I) 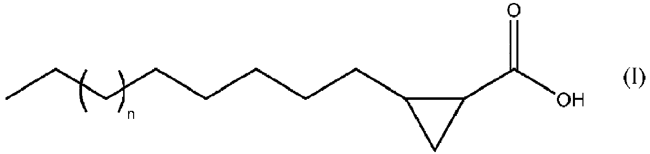 wobei n für 1 oder 2 steht, wobei das Gewichtsverhältnis von cis-Diastereoisomeren zu trans-Diastereoisomeren im Bereich zwischen 90: 10 und 99,5:0,5 liegt, und umfassend mindestens 10 Gew.-% cis-(1S,2R)-Diastereoisomer, wobei sich der Prozentsatz auf das Gesamtgewicht der Stoffzusammensetzung bezieht.

2. Stoffzusammensetzung nach Anspruch 1, wobei die Stoffzusammensetzung Folgendes umfasst:
a) 90 bis 99,5 Gew.-% cis-2-Octylcyclopropancarbonsäure;
b) 0,5 % bis 10 Gew.-% trans-2-Octylcyclopropancarbonsäure; wobei sich der Prozentsatz auf das Gesamtgewicht der Stoffzusammensetzung bezieht.

3. Stoffzusammensetzung nach einem der Ansprüche 1 bis 2, wobei die Stoffzusammensetzung mindestens 45 Gew.-% cis-(1S,2R)-2-Octylcyclopropancarbonsäure umfasst, wobei sich der Prozentsatz auf das Gesamtgewicht der Stoffzusammensetzung bezieht.

4. Stoffzusammensetzung nach einem der Ansprüche 1 bis 3, wobei die Stoffzusammensetzung Folgendes umfasst:
a) 45 bis 99,5 Gew.-% cis-(1S,2R)-2-Octylcyclopropancarbonsäure;
b) höchstens 49,75 Gew.-% cis-(1R,2S)-2-Octylcyclopropancarbonsäure;
c) höchstens 10 Gew.-% trans-(1S,2S)-2-Octylcyclopropancarbonsäure;
d) höchstens 10 Gew.-% trans-(1R,2R)-2-Octylcyclopropancarbonsäure, wobei sich der Prozentsatz auf das Gesamtgewicht der Stoffzusammensetzung bezieht und wobei das Gewichtsverhältnis von cis-Diastereoisomeren zu trans-Diastereoisomeren im Bereich zwischen 90: 10 und 99,5:0,5 liegt.

5. Stoffzusammensetzung nach einem der Ansprüche 1 bis 4, wobei die Stoffzusammensetzung Folgendes umfasst:
a) 47,5 bis 99 Gew.-% cis-(1S,2R)-2-Octylcyclopropancarbonsäure;
b) höchstens 49,5 Gew.-% cis-(1R,2S)-2-Octylcyclopropancarbonsäure;
c) höchstens 5 Gew.-% trans-(1S,2S)-2-Octylcyclopropancarbonsäure;
d) höchstens 5 Gew.-% trans-(1R,2R)-2-Octylcyclopropancarbonsäure.

6. Stoffzusammensetzung nach einem der Ansprüche 1 bis 5, wobei das Gewichtsverhältnis von cis-Diastereoisomeren zu trans-Diastereoisomeren im Bereich zwischen 95:5 und 99:1 liegt.

7. Stoffzusammensetzung nach einem der Ansprüche 1 bis 6, wobei cis-2-Octylcyclopropancarbonsäure und trans-2-Octylcyclopropancarbonsäure in einer racemischen Form vorliegen.

8. Stoffzusammensetzung nach einem der Ansprüche 1 bis 7, wobei die Stoffzusammensetzung Folgendes umfasst:
a) 47,5 bis 49,75 Gew.-% cis-(1S,2R)-2-Octylcyclopropancarbonsäure;
b) 47,5 bis 49,75 Gew.-% cis-(1R,2S)-2-Octylcyclopropancarbonsäure;
c) 0,5 % bis 2,5 Gew.-% trans-(1S,2S)-2-Octylcyclopropancarbonsäure;
d) 0,5 % bis 2,5 Gew.-% trans-(1R,2R)-2-Octylcyclopropancarbonsäure.

9. Verfahren zum Verleihen, Verstärken, Verbessern oder Modifizieren der Geruchseigenschaften einer Parfümierungszusammensetzung oder eines parfümierten Artikels oder einer Oberfläche, wobei das Verfahren die Zugabe einer wirksamen Menge einer wie in einem der Ansprüche 1 bis 8 definierten Stoffzusammensetzung zu der Zusammensetzung oder dem parfümierten Artikel umfasst.

10. Verwendung einer Stoffzusammensetzung gemäß einem der Ansprüche 1 bis 8 als Parfümierungsbestandteil.

11. Parfümierungszusammensetzung, umfassend
i) eine wie in einem der Ansprüche 1 bis 8 definierte Stoffzusammensetzung,
ii) mindestens einen aus der aus einem Parfümierungsträger und einer Parfümierungsgrundlage bestehenden Gruppe ausgewählten Bestandteil und
iii) gegebenenfalls mindestens ein Parfümierungsadjuvans.

12. Parfümiertes Konsumprodukt, das eine Stoffzusammensetzung gemäß einem der Ansprüche 1 bis 8 oder eine Parfümierungszusammensetzung gemäß Anspruch 11 umfasst.

13. Parfümiertes Konsumprodukt nach Anspruch 12, wobei es sich bei dem Parfümerie-Konsumprodukt um ein Parfüm, ein Textilpflegeprodukt, ein Körperpflegeprodukt, eine Kosmetikzubereitung, ein Hautpflegeprodukt, ein Luftpflegeprodukt oder ein Haushaltspflegeprodukt handelt.

14. Parfümiertes Konsumprodukt nach Anspruch 13, wobei es sich bei dem Parfümerie-Konsumprodukt um ein ein feines Parfüm, ein Parfümwasser bzw. Eau de Parfum, ein Kölnisch Wasser, eine Rasierlotion oder eine After-Shave-Lotion, ein flüssiges, ein Pod- oder festes Waschmittel oder eine Waschmitteltablette, einen Weichspüler, einen flüssigen oder festen Duftverstärker, ein Trocknertuch, ein Auffrischer für Textilien, ein Bügelwasser, ein Papier, ein Bleichmittel, einen Teppichreiniger, ein Produkt zur Pflege von Vorhängen, ein Shampoo, einen Conditioner, der im Haar verbleibt oder ausgespült wird, ein Färbepräparat, ein Farbpflegeprodukt, ein Haarformungsprodukt, ein Zahnpflegeprodukt, ein Desinfektionsmittel, ein Intimpflegeprodukt, ein Haarspray, eine Hautcreme oder -lotion, eine Abdeckcreme, ein Deodorant oder Antitranspirant, einen Haarentferner, ein Bräunungs-, Sonn- oder After-Sun-Produkt, ein Nagelprodukt, ein Hautreinigungsmittel, ein Make-up, eine parfümierte Seife, einen Dusch- oder Badeschaum, ein Dusch- oder Badeöl oder -gel, ein Fuß-/Handpflegeprodukt, ein Hygieneprodukt, einen Lufterfrischer, einen gebrauchsfertigen pulverförmigen Lufterfrischer, einen Schimmelentferner, ein Möbelpflegemittel, ein Wischtuch, ein Geschirrspülmittel oder Reinigungsmittel für harte Oberflächen, ein Lederpflegeprodukt oder ein Autopflegeprodukt handelt.

## Revendications

1. Composition de matière comprenant un composé de formule (1) 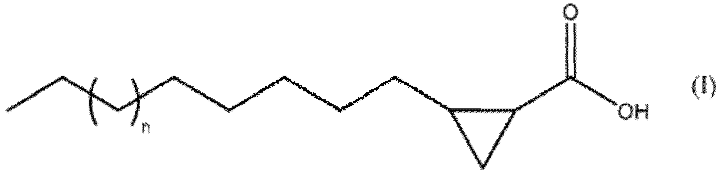 n étant 1 ou 2 ; le rapport en poids des diastéréoisomères cis sur les diastéréoisomères trans étant compris dans la plage entre 90 : 10 et 99,5 : 0,5 et comprenant au moins 10 % p/p de diastéréoisomère cis-(1S,2R), le pourcentage étant relatif au poids total de la composition de matière.

2. Composition de matière selon la revendication 1, la composition de matière comprenant :
a) de 90 à 99,5 % p/p d'acide cis-2-octylcyclopropanecarboxylique ;
b) de 0,5 % à 10 % p/p d'acide trans-2-octylcyclopropanecarboxylique ;
le pourcentage étant relatif au poids total de la composition de matière.

3. Composition de matière selon l'une quelconque des revendications 1 à 2, la composition de matière comprenant au moins 45 % p/p d'acide cis-(1S,2R)-2-octylcyclopropanecarboxylique, le pourcentage étant relatif au poids total de la composition de matière.

4. Composition de matière selon l'une quelconque des revendications 1 à 3, la composition de matière comprenant :
a) de 45 à 99,5 % p/p d'acide cis-(1S,2R)-2-octylcyclopropanecarboxylique ;
b) au plus 49,75 % p/p d'acide cis-(1R,2S)-2-octylcyclopropanecarboxylique ;
c) au plus 10 % p/p d'acide trans-(1S,2S)-2-octylcyclopropanecarboxylique ;
d) au plus 10 % p/p d'acide trans-(1R,2R)-2-octylcyclopropanecarboxylique ;
le pourcentage étant relatif au poids total de la composition de matière ; et
le rapport en poids des diastéréoisomères cis sur les diastéréoisomères trans étant compris dans la plage entre 90 : 10 et 99,5 : 0,5.

5. Composition de matière selon l'une quelconque des revendications 1 à 4, la composition de matière comprenant :
a) de 47,5 à 99 % p/p d'acide cis-(1S,2R) -2-octylcyclopropanecarboxylique ;
b) au plus 49,5 % p/p d'acide cis- (1R,2S)-2-octylcyclopropanecarboxylique ;
c) au plus 5 % p/p d'acide trans-(1S,2S)-2-octylcyclopropanecarboxylique ;
d) au plus 5 % p/p d'acide trans-(1R,2R)-2-octylcyclopropanecarboxylique.

6. Composition de matière selon l'une quelconque des revendications 1 à 5, le rapport en poids des diastéréoisomères cis sur les diastéréoisomères trans étant compris dans la plage entre 95 : 5 et 99 : 1.

7. Composition de matière selon l'une quelconque des revendications 1 à 6, l'acide cis-2-octylcyclopropanecarboxylique et l'acide trans-2-octylcyclopropanecarboxylique étant sous une forme racémique.

8. Composition de matière selon l'une quelconque des revendications 1 à 7, la composition de matière comprenant :
a) de 47,5 à 49,75 % p/p d'acide cis-(1S,2R)-2-octylcyclopropanecarboxylique ;
b) de 47,5 à 49,75 % p/p d'acide cis-(1R,2S)-2-octylcyclopropanecarboxylique ;
c) de 0,5 % à 2,5 % p/p d'acide trans-(1S,2S)-2-octylcyclopropanecarboxylique ;
d) de 0,5 % à 2,5 % p/p d'acide trans-(1R,2R)-2-octylcyclopropanecarboxylique.

9. Procédé pour conférer, augmenter, améliorer ou modifier les propriétés odorantes d'une composition parfumante ou d'un article parfumé ou d'une surface, lequel procédé comprenant un ajout à ladite composition ou audit article parfumé d'une quantité efficace d'une composition de matière telle que définie dans l'une quelconque des revendications 1 à 8.

10. Utilisation en tant qu'ingrédient parfumant d'une composition de matière telle que définie dans l'une quelconque des revendications 1 à 8.

11. Composition parfumante comprenant
i) une composition de matière, telle que définie dans l'une quelconque des revendications 1 à 8 ;
ii) au moins un ingrédient choisi dans le groupe constitué par un support de parfumerie et une base de parfumerie ; et
iii) éventuellement au moins un adjuvant de parfumerie.

12. Produit de consommation parfumé comprenant une composition de matière telle que définie dans l'une quelconque des revendications 1 à 8 ou une composition parfumante telle que définie dans la revendication 11.

13. Produit de consommation parfumé selon la revendication 12, le produit de consommation de parfumerie étant un parfum, un produit d'entretien de tissus, un produit de soin personnel, une préparation cosmétique, un produit de soin de la peau, un produit d'assainissement de l'air ou un produit d'entretien ménager.

14. Produit de consommation parfumé selon la revendication 13, le produit de consommation de parfumerie étant un parfum fin, une eau de toilette ou une eau de parfum, une eau de Cologne, une lotion de rasage ou d'après-rasage, un liquide, une capsule, ou un détergent ou comprimé solide, un assouplissant pour tissus, un rehausseur de parfum liquide ou solide, une feuille pour séchoir, un rafraîchisseur de tissus, une eau de repassage, un papier, un agent de blanchiment, un agent de nettoyage pour tapis, un produit d'entretien de rideaux, un shampooing, un après-shampooing sans rinçage ou à rincer, une préparation de coloration, un produit d'entretien de couleurs, un produit de coiffage capillaire, un produit de soin dentaire, un désinfectant, un produit d'hygiène intime, un spray capillaire, une crème ou lotion pour la peau, une crème fondante, un déodorant ou antitranspirant, un agent d'élimination de cheveux, un produit de bronzage ou solaire ou aprèssoleil, un produit pour les ongles, un agent de nettoyage de la peau, un maquillage, un savon parfumé, une mousse de douche ou de bain, une huile ou un gel, un produit de soin pour les pieds/les mains, un produit hygiénique, un désodorisant, un désodorisant en poudre « prêt à l'emploi », un agent d'élimination de moisissures, un produit d'entretien de meubles, une lingette, un détergent pour vaisselle ou un détergent pour surface dure, un produit d'entretien du cuir, un produit d'entretien d'automobile.
